# EUROPEAN PATENT SPECIFICATION

(11) **EP 3 387 923 B1**
(45) Date of publication and mention of the grant of the patent: **29.01.2020**
(21) Application number: 17875058.4
(22) Date of filing: 07.09.2017
(51) Int. Cl.: A24F 47/00, G01K 13/02

(54) **ELECTRONIC CIGARETTE DEVICE AND METHOD FOR CALCULATING PUFF COUNT**
ELEKTRONISCHE ZIGARETTENVORRICHTUNG UND VERFAHREN ZUR BERECHNUNG DER ANZAHL DER ZÜGE
DISPOSITIF DE CIGARETTE ÉLECTRONIQUE ET PROCÉDÉ DE CALCUL DU NOMBRE DE BOUFFÉES

(30) Priority: 16.02.2017 CN 201710084309; 13.07.2017 CN 201710571669
(43) Date of publication of application: 17.10.2018
(73) Proprietor: SMISS Technology Co., Ltd, Shenzhen Guangdong (CN)
(72) Inventor: CHEN, Jiatai, Guangming New District Shenzhen, Guangdong (CN); SONG, Hailong, Guangming New District Shenzhen, Guangdong (CN); CHEN, Shikai, Guangming New District Shenzhen, Guangdong (CN)
(74) Representative: Zaboliene, Reda
(86) International application number: PCT/CN2017/100813
(87) International publication number: WO 2018/149117

(56) References cited:
- EP-A1- 2 907 398
- CN-A- 103 034 689
- CN-A- 103 734 915
- CN-A- 105 146 756
- CN-U- 202 792 097
- CN-U- 204 483 016
- CN-U- 204 519 364
- CN-U- 204 670 381
- CN-U- 205 161 888
- GB-A- 2 507 104
- US-A1- 2012 260 926
- US-A1- 2013 284 192
- US-A1- 2016 227 842

## Description

### BACKGROUND OF THE INVENTION

### Field of the Invention

The present application relates to the field of cigarette technologies, and in particular, to an electronic cigarette and a method for detecting the number of times of inhaling of an electronic cigarette.

### Related Arts

In a conventional method for detecting the number of times of inhaling of an electronic cigarette, a microphone is usually disposed at an air entrance of the cigarette, and a negative pressure generated during inhaling is detected by using the microphone, to detect the number of times of inhaling. However, when the negative pressure generated during inhaling is detected by using the microphone, it can be implemented when a sufficient airflow passes, and a relatively large negative pressure value is generated, and is applicable to an electronic cigarette with a relatively large airflow passing amount.

However, for an electronic cigarette that bakes a new tobacco, due to reasons of the new tobacco and a structure of an airflow path, an airflow amount during inhaling is relatively small, and the microphone can hardly sense a negative pressure generated during inhaling. Consequently, detection of the number of times of inhaling can easily be inaccurate.

US 2016/0227842A1 disclosed an electronic cigarette including an electronic cigarette body is provided. The electronic cigarette body is provided with an atomizer assembly configured to atomize nicotine liquid and a battery assembly configured to power the electronic cigarette body. A communication assembly is disposed between the atomizer assembly and the battery assembly and is provided with a first connecting end detachably connected to the battery assembly, a second connecting end detachably connected to the atomizer assembly, and a conductive module configured to connect the battery assembly to the atomizer assembly. The communication assembly is further provided with a microcontroller configured to record smoking information of the electronic cigarette according to an electrical signal of the conductive module and control operation of modules of the communication assembly, and a wireless communication module configured to establish communication connection with the external terminal.

GB2507104A disclosed an electronic inhalation device comprises a mouthpiece 2 and a control unit 24, the control unit 24 comprising a power cell 22 and a computer 20, where the computer 20 comprises a computer processor, a memory and an input-output means; wherein the device further comprises a transmitter 18 connected to the computer 20 and the computer is configured in use to collect and store use data relating to a user's use of the device in the computer memory and transmit the use data. Preferably, the use data is transmitted wirelessly to a receiver, which may be provided on a smart phone for example. The transmitter preferably comprises audio signalling means and transmits the use data by sound. The receiver may then be provided as a microphone which picks up the sound signal, preferably transmitted at a frequency to avoid background noise.

### SUMMARY

In view of the above, it is necessary to provide an electronic cigarette capable of effectively improving detection accuracy of the number of times of inhaling and a method for detecting the number of times of inhaling of an electronic cigarette.

An electronic cigarette, including:
a cigarette body, where the cigarette body includes a cylinder, the cylinder has an accommodating cavity, the cigarette body is further provided with an air inlet passage, the air inlet passage has an air inlet, and the air inlet passage is in communication with the accommodating cavity to form an airflow passage;
a temperature sensing element, where the temperature sensing element includes a temperature sensing end, the temperature sensing end is disposed within the airflow passage, when an inhaling action is generated, outside air enters the air inlet passage from the air inlet and flows into the accommodating cavity, an airflow flowing within the airflow passage is formed, the temperature sensing end of the temperature sensing element is used to detect a temperature of the airflow when inhaling is performed and when inhaling is not performed to learn of change of the temperature and obtain the number of times of inhaling according to the change of the temperature; and
a circuit board, where the circuit board is electrically connected to the temperature sensing element, and the circuit board is electrically connected to the cigarette body.

After the electronic cigarette is electrified, heat is generated within the cigarette body, and the heat heats air within the cigarette body. Because the temperature sensing end of the temperature sensing element is located within the airflow passage, the heat generated within the cigarette body radiates to the temperature sensing end of the temperature sensing element. When the inhaling action is generated, outside air enters the air inlet passage from the air inlet and flows into the accommodating cavity, and the airflow flowing within the airflow passage is formed. Therefore, when inhaling is performed and when inhaling is not performed, temperature values detected by the temperature sensing end are different to learn of change of the temperature. The circuit board obtains the number of times of inhaling according to the change of the temperature. Therefore, to obtain the number of times of inhaling, a negative pressure does not need to be sensed, but only the temperature sensing end of the temperature sensor needs to detect the change of the temperature. Therefore, detection accuracy of the number of times of inhaling can be effectively improved.

A method for detecting the number of times of inhaling of an electronic cigarette, including:
electrifying a cigarette body, to preheat air within the cigarette body until a temperature within the cigarette body reaches a preset temperature value, where
a temperature value detected by a temperature sensing end of a temperature sensing element is a first temperature value when inhaling is not performed;
when an inhaling action is generated, outside air enters a cylinder through an air inlet via an air inlet passage, and an airflow flowing within an airflow passage is formed; when the airflow flows through the temperature sensing end of the temperature sensing element, a temperature value detected by the temperature sensing end is a second temperature value, and there is a temperature difference between the second temperature value and the first temperature value; and
a circuit board receiving the temperature difference generated between the first temperature value and the second temperature value that are detected by the temperature sensing element and converting the temperature difference into an inductive signal, and detecting the inductive signal to obtain the number of times of inhaling.

In the method for detecting the number of times of inhaling of an electronic cigarette, the cigarette body is electrified, to preheat air within the cigarette body until the temperature within the cigarette body reaches the preset temperature value. The temperature value detected by the temperature sensing end of the temperature sensing element is the first temperature value when inhaling is not performed. When the inhaling action is generated, outside air enters the cylinder through the air inlet via the air inlet passage, and the airflow flowing within the airflow passage is formed; when the airflow flows through the temperature sensing end of the temperature sensing element, the temperature value detected by the temperature sensing end is the second temperature value, and there is a temperature difference between the second temperature value and the first temperature value. The circuit board receives the temperature difference generated between the first temperature value and the second temperature value that are detected by the temperature sensing element and converts the temperature difference into the inductive signal, and detects the inductive signal to obtain the number of times of inhaling. Therefore, to obtain the number of times of inhaling, a negative pressure does not need to be sensed, but only the temperature sensing end of the temperature sensor needs to detect the change of the temperature. Therefore, detection accuracy of the number of times of inhaling can be effectively improved.

### BRIEF DESCRIPTION OF THE DRAWINGS

To describe technical solutions in the embodiments of the present application or the prior art more clearly, the following briefly introduces the accompanying drawings required for describing the embodiments or the prior art. Apparently, the accompanying drawings in the following descriptions show merely some embodiments of the present application, and a person of ordinary skill in the art may still derive drawings of other embodiments from these accompanying drawings without creative efforts.
FIG. 1 is a schematic structural diagram of an electronic cigarette according to a first implementation;
FIG. 2 is a sectional view of the electronic cigarette shown in FIG. 1;
FIG. 3 is a schematic structural diagram of an electronic cigarette according to a second implementation;
FIG. 4 is a sectional view of the electronic cigarette shown in FIG.3;
FIG. 5 is a schematic structural diagram of an electronic cigarette according to a third implementation;
FIG. 6 is a sectional view of the electronic cigarette shown in FIG. 5;

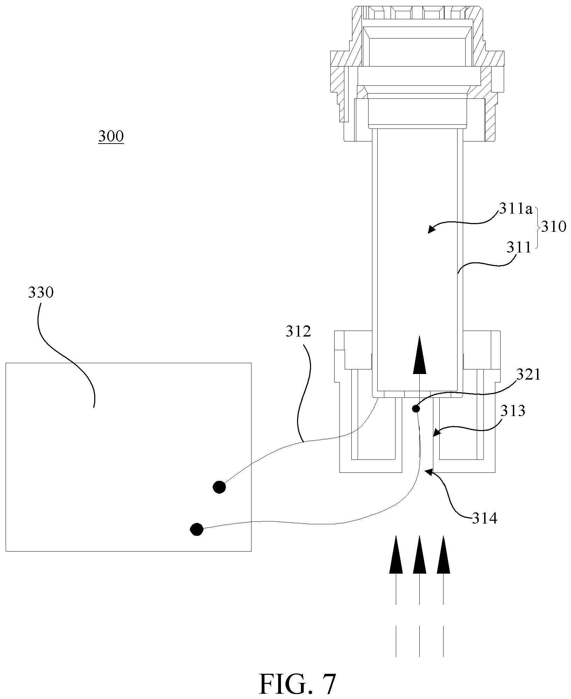

FIG. 7 is a sectional view of an electronic cigarette according to a fourth implementation;
FIG. 8 is a sectional view of an electronic cigarette according to a fifth implementation;
FIG. 9 is a schematic structural diagram of an electronic cigarette according to a sixth implementation;
FIG. 10 is a sectional view of the electronic cigarette shown in FIG. 9;
FIG. 11 is a schematic structural diagram of an electronic cigarette according to a seventh implementation;
FIG. 12 is a sectional view of the electronic cigarette shown in FIG. 11;
FIG. 13 is a schematic structural diagram of an electronic cigarette according to an eighth implementation;
FIG. 14 is a sectional view of the electronic cigarette shown in FIG. 13;
FIG. 15 is a sectional view of an electronic cigarette according to a tenth implementation;
FIG. 16 is a sectional view of an electronic cigarette according to a ninth implementation; and
FIG. 17 is a schematic flowchart of a method for detecting the number of times of inhaling of an electronic cigarette according to an implementation.

### DETAILED DESCRIPTION

To make the above objectives, features, and advantages of the present application more obvious and easier to understand, the following describes specific implementations of the present application in detail with reference to the accompanying drawings. In the following descriptions, many specific details are stated to fully understand the present application. However, the present application can be implemented in many other manners different from those described herein. A person skilled in the art may make similar improvement without violating content of the present application. Therefore, the present application is not limited by specific implementations disclosed below.

It should be noted that when an element is referred to as being "fixed" on another element, the element may be directly on another element or there may be a middle element. When an element is considered to be "connected" to another element, the element may be directly connected to another element or there may be a middle element at the same time. Terms "vertical", "horizontal", "left" and "right" used in this text and similar expressions are merely for the purpose of illustration and do not indicate unique implementations.

Unless otherwise defined, all technical and scientific terms used in this text are the same as meanings that a person skilled in the art usually understood. Terms used in the specification of the present application in this text are merely for the purpose of describing specific embodiments and are not aimed to limit the present application. Various technical features in the following embodiments may be combined randomly. For ease of description, possible combinations of various technical features in the following embodiments are not all described. However, the combinations of the technical features should be considered as falling within the scope recorded in this specification provided that the combinations of the technical features are compatible with each other.

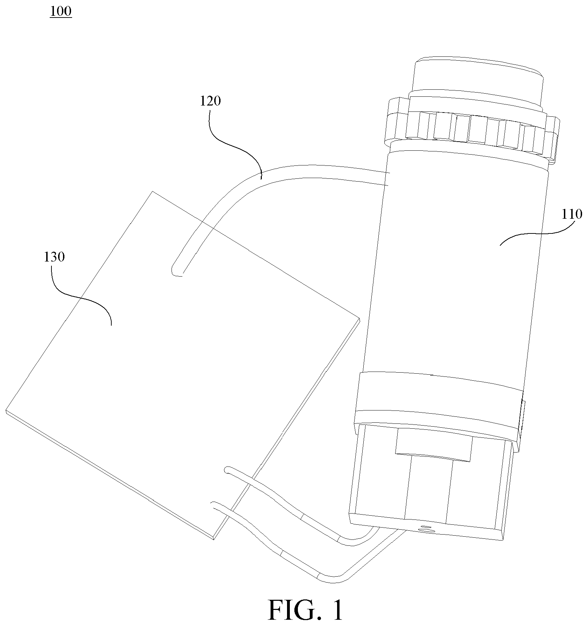

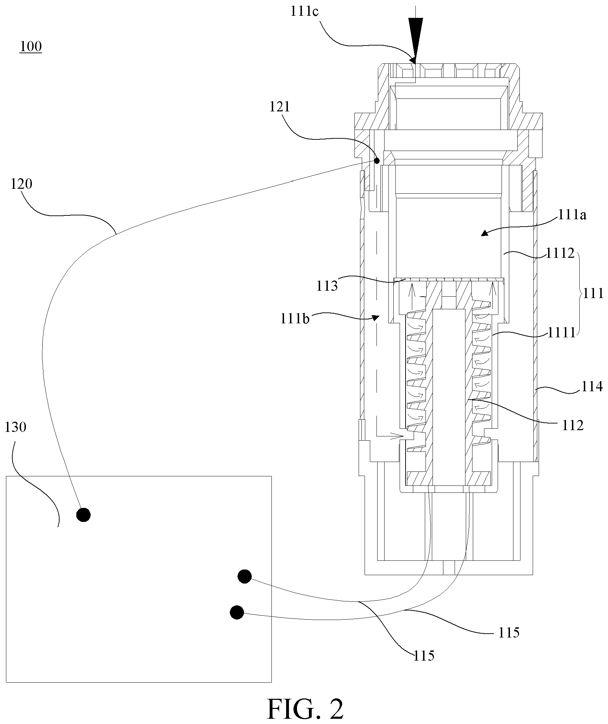

Referring to FIG. 1 and FIG. 2, an electronic cigarette 100 in a first implementation includes a cigarette body 110, a temperature sensing element 120, and a circuit board 130. The cigarette body 110 and the temperature sensing element 120 are separately electrically connected to the circuit board 130.

Specifically, in the first implementation, the cigarette body 110 includes a cylinder 111, a heating component 112, a filter screen 113, and a sleeve 114. The cylinder 111 has an accommodating cavity 111a, and the cylinder 111 is divided by the filter screen 113 into a first cylinder 1111 and a second cylinder 1112. That is, the filter screen 113 divides the accommodating cavity 111a into an upper cavity and a lower cavity that are in communication. The lower cavity is formed within the first cylinder 1111, and the upper cavity is formed within the second cylinder 1112. The heating component 112 is disposed within the first cylinder 1111, and the second cylinder 1112 is used to place a tobacco product. The filter screen 113 is mainly used to filter scrapes and the like generated by the tobacco product disposed within the second cylinder 1112, and prevent the scrapes from falling on the heating component 112.

The heating component 112 is electrically connected to the circuit board 130. Specifically, the heating component 112 is electrically connected to the circuit board 130 through a conductive wire 115. After being electrified, the heating component 112 generates heat, and the heat is used to heat air within the cigarette body 110. The cylinder may be a heating cylinder having a heating function. In this case, the heating cylinder and the heating component together generate heat to heat and bake the tobacco product. Certainly, in other implementations, the cylinder may not have a heating function but have a thermally conductive function.

The sleeve 114 is sleeved outside the cylinder 111, an air inlet passage 111b is formed between an inner wall of the sleeve 114 and an outer wall of the cylinder 111, and the air inlet passage 111b has an air inlet 111c. The air inlet passage 111b and the accommodating cavity 111a are in communication to form an airflow passage. During inhaling, outside cold air enters the air inlet passage 111b through the air inlet 111c under an inhaling force and enters the accommodating cavity 111a, and an airflow flowing within the airflow passage is formed (as shown in a dotted arrow direction in FIG. 2).

Specifically, in the first implementation, the heating component 112 includes a thermally conductive rod and a heating wire. The heating wire is winded on the thermally conductive rod. Two ends of the heating wire are separately electrically connected to the circuit board 130. The cylinder 111 may be made of an insulating and easily thermally conductive material. In this case, the cylinder 111 mainly conducts heat generated by the heating wire. For example, the cylinder 111 may be an easily thermally conductive metal, ceramic, or another medium. The cylinder 111 may also be a thin aluminum material after insulation treatment or the like. The circuit board 130 may be a printed circuit board assembly (PCBA) board.

The material of the filter screen 113 may be an easily thermally conductive and easily cleaned material, for example, a metal and stainless steel. By means of close fitting of the thermally conductive rod and the filter screen 113, heat generated by the heating wire can be fast conducted onto the filter screen 113 through the thermally conductive rod, and the filter screen 113 is in direct contact with the tobacco product. Therefore, filter screen 113 can heat the tobacco product, thereby improving heating efficiency.

A spiral groove is provided on an outer side wall of the thermally conductive rod. The spiral groove extends along an axial direction of the thermally conductive rod. The heating wire is winded on the thermally conductive rod in a spiral shape along the spiral groove. The heating wire, the outer side wall of the thermally conductive rod, a side wall of the spiral groove, and an inner side wall of the cylinder 111 together form a flow guide passage through encircling. The height of the side wall of the spiral groove is greater than a diameter size of the heating wire, to ensure the size of the flow guide passage.

Because the heating wire is winded on the outer side wall of the thermally conductive rod in a spiral shape along the spiral groove, an airflow is directly heated by using the heating wire, heat generated by the heating wire may also make the temperature of the thermally conductive rod rise. After the temperature of the thermally conductive rod rises, the airflow may also be heated. The heating wire is electrically connected to the circuit board 130 by using a wire.

Certainly, in other implementations, a heating manner may also be an inductive heating manner. Correspondingly, the heating component is an inductive heating structure.

The temperature sensing element 120 includes a temperature sensing end 121, the temperature sensing end 121 is disposed within the airflow passage, and the temperature sensing element 120 is electrically connected to the circuit board 130. The temperature sensing element 120 may be a temperature sensor, a negative temperature coefficient (NTC) thermistor, or a thermocouple. A hole is provided on the side wall of the sleeve 114. The temperature sensing end 121 of the temperature sensing element 120 extends into the airflow passage through the hole, and then the hole is sealed.

When an inhaling action is generated, outside air enters the air inlet passage 111b from the air inlet 111c and flows into the accommodating cavity 111a, and an airflow flowing within the airflow passage is formed. The temperature sensing end 121 of the temperature sensing element 120 is used to detect a temperature within the airflow passage when inhaling is performed and when inhaling is not performed to learn of change of the temperature and obtain the number of times of inhaling according to the change of the temperature.

Specifically, in the first implementation, the temperature sensing end 121 of the temperature sensing element 120 is located at a position close to an air source, so that a detected temperature difference is relatively large, to improve accuracy of detection of the number of times of inhaling. Specifically, the temperature sensing end 121 of the temperature sensing element 120 is located within the air inlet passage 111b and is close to the air inlet 111c.

In the foregoing first implementation, after the electronic cigarette 100 is electrified, the heating component 112 generates heat, and the heat preheats air within the cigarette body 110. Because the temperature sensing end 121 of the temperature sensing element 120 is located within the air inlet passage 111b, the heat generated by the heating component 112 radiates to the temperature sensing end 121 of the temperature sensing element 120. When inhaling is not performed, the temperature after preheating detected by the temperature sensing element 120 is a first temperature value. In this case, the temperature detected by the temperature sensing element 120 is higher than the temperature of the outside air.

When an inhaling action is generated, outside air enters the air inlet passage 111b from the air inlet 111c and flows into the accommodating cavity 111a, and an airflow flowing within the airflow passage is formed. Because the temperature sensing element 120 is close to the air inlet 111c, in this case, a temperature value detected by the temperature sensing element 120 is a second temperature value, the second temperature value is equivalent to a temperature value of cold air from the outside, and the second temperature value is lower than the first temperature value. Therefore, when inhaling is performed and when inhaling is not performed, the temperature sensing end 121 detects different temperature values to obtain change of the temperature. The circuit board 130 obtains the number of times of inhaling according to the change of the temperature. Therefore, to obtain the number of times of inhaling, a negative pressure does not need to be sensed, but only the temperature sensing end 121 of the temperature sensor needs to detect the change of the temperature. Therefore, detection accuracy of the number of times of inhaling can be effectively improved.

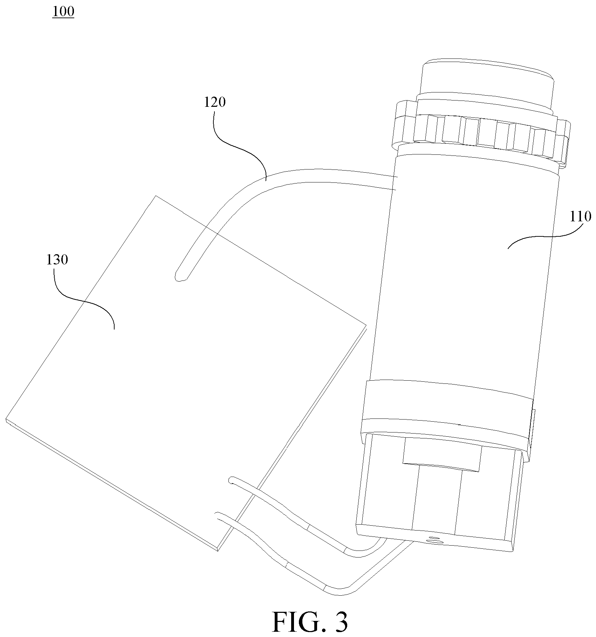

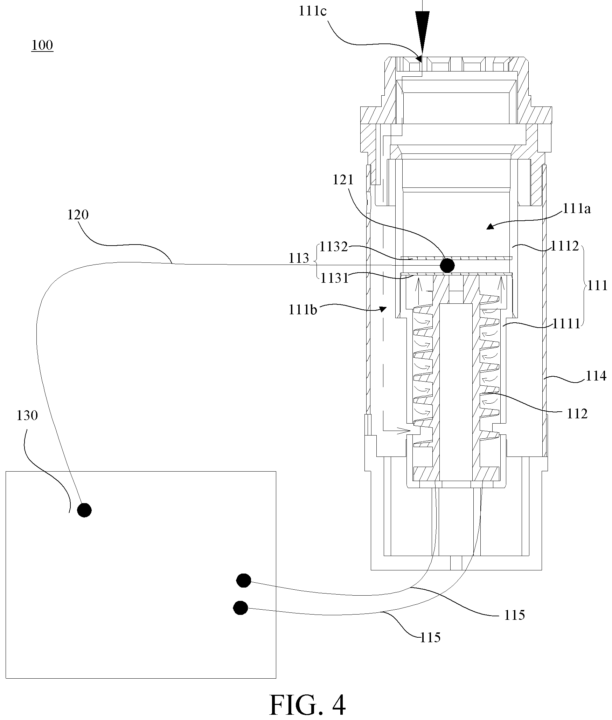

Certainly, referring to FIG. 3 and FIG. 4, in a second implementation, the temperature sensing end 121 of the temperature sensing element 120 is located at a position close to a heating source. Specifically, the temperature sensing end 121 of the temperature sensing element 120 is located within the cylinder 111 and is located at a position, close to the heating component 112, within the second cylinder 1112. Specifically, there are two filter screens 113. The two filter screens 113 are disposed at an interval and are respectively a first filter screen 1131 and a second filter screen 1132. The first filter screen 1131 is in direct contact with the heating component 112. The second filter screen 1132 is located within the second cylinder 1112. The temperature sensing end 121 of the temperature sensing element 120 is located between the first filter screen 1131 and the second filter screen 1132.

Certainly, in other implementations, there may also be only one filter screen 113. The temperature sensing end 121 of the temperature sensing element 120 is directly located within the second cylinder 1112 and is close to the heating component 112.

In the foregoing second implementation, after the electronic cigarette 100 is electrified, the heating component 112 generates heat, and the heat preheats air within the cigarette body 110, Because the temperature sensing end 121 of the temperature sensing element 120 is located within the cylinder 111 and is located between the first filter screen 1131 and the second filter screen 1132, the heat generated by the heating component 112 radiates to the temperature sensing end 121 of the temperature sensing element 120. When inhaling is not performed, the temperature after preheating detected by the temperature sensing element 120 is a first temperature value. In this case, the temperature detected by the temperature sensing element 120 is lower than the temperature of the heating component 112.

When an inhaling action is generated, outside air enters the air inlet passage 111b from the air inlet 111c and flows into the accommodating cavity 111a, and an airflow flowing within the airflow passage is formed. Because the temperature sensing element 120 is located at a position, close to the heating component 112, within the second cylinder 1112, in this case, a temperature value detected by the temperature sensing element 120 is a second temperature value, the second temperature value is equivalent to a temperature value of air within the first cylinder 1111, and the second temperature value is higher than the first temperature value. Therefore, when inhaling is performed and when inhaling is not performed, the temperature sensing end 121 detects different temperature values to obtain change of the temperature. The circuit board 130 obtains the number of times of inhaling according to the change of the temperature. Therefore, to obtain the number of times of inhaling, a negative pressure does not need to be sensed, but only the temperature sensing end 121 of the temperature sensor needs to detect the change of the temperature. Therefore, detection accuracy of the number of times of inhaling can be effectively improved.

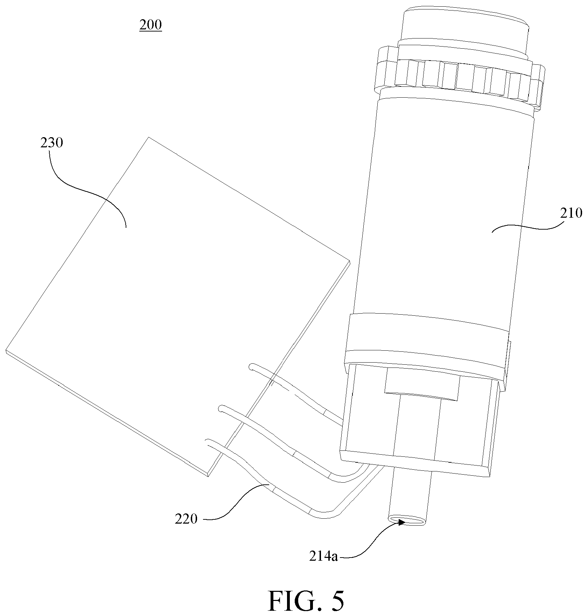

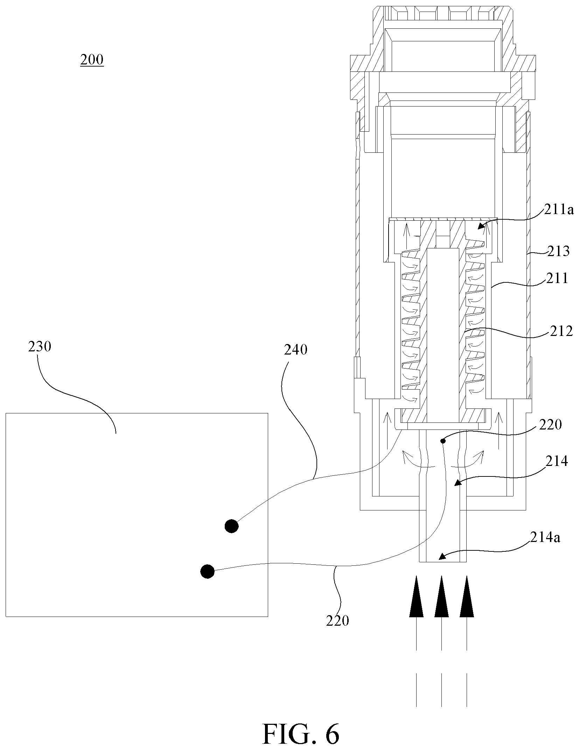

Referring to FIG. 5 and FIG. 6, an electronic cigarette 200 in a third implementation includes a cigarette body 210, a temperature sensing element 220, and a circuit board 230. The cigarette body 210 and the temperature sensing element 220 are both electrically connected to the circuit board 230.

Specifically, the cigarette body 210 includes a cylinder 211, a heating component 212, and a casing 213. The cylinder 211 has an accommodating cavity 211a, the heating component 212 is accommodated within the accommodating cavity 211a of the cylinder 211, and the heating component 212 is electrically connected to the circuit board 230 by using a conductive wire 240. After being electrified, the heating component 212 generates heat, and the heat is used to heat air within the cigarette body 210.

The casing 213 is sleeved outside the cylinder 211, and an air inlet passage 214 is located at one end, provided with the heating component 212, of the cylinder 211. That is, the air inlet passage 214 is located at the bottom of the cylinder 211. The air inlet passage 214 has an air inlet 214a, and the air inlet passage 214 and the accommodating cavity 211a are in communication to form an airflow passage. During inhaling, outside cold air enters the air inlet passage 214 through the air inlet 214a under an inhaling force and enters the accommodating cavity 211a, and an airflow flowing within the airflow passage is formed (as shown in an arrow direction in FIG. 6).

Specifically, in the third implementation, the heating component 212 includes a thermally conductive rod and a heating wire. The heating wire is winded on the thermally conductive rod. Two ends of the heating wire are separately electrically connected to the circuit board 230. The cylinder 211 may be made of an insulating and easily thermally conductive material. In this case, the cylinder 211 mainly conducts heat generated by the heating wire. For example, the cylinder 211 may be an easily thermally conductive metal, ceramic, or another medium. The cylinder 211 may also be a thin aluminum material after insulation treatment or the like. The circuit board 230 may be a printed circuit board assembly (PCBA) board.

A spiral groove is provided on an outer side wall of the thermally conductive rod. The spiral groove extends along an axial direction of the thermally conductive rod. The heating wire is winded on the thermally conductive rod in a spiral shape along the spiral groove. The heating wire, the outer side wall of the thermally conductive rod, a side wall of the spiral groove, and an inner side wall of the cylinder 211 together form a flow guide passage through encircling. The height of the side wall of the spiral groove is greater than a diameter size of the heating wire, to ensure the size of the flow guide passage.

Because the heating wire is winded on the outer side wall of the thermally conductive rod in a spiral shape along the spiral groove, an airflow is directly heated by using the heating wire, heat generated by the heating wire may also make the temperature of the thermally conductive rod rise. After the temperature of the thermally conductive rod rises, the airflow may also be heated. The heating wire is electrically connected to the circuit board 230 by using a wire.

The temperature sensing element 220 includes a temperature sensing end 221, the temperature sensing end 221 is disposed within the airflow passage, and the temperature sensing element 220 is electrically connected to the circuit board 230. Specifically, the temperature sensing end 221 of the temperature sensing element 220 is disposed within the air inlet passage 214. Therefore, the temperature sensing end 221 of the temperature sensing element 220 is relatively close to the air inlet 214a and is also relatively close to the heating component 212. The temperature sensing element 220 may be a temperature sensor, a negative temperature coefficient (NTC) thermistor, or a thermocouple.

In the foregoing third implementation, after the electronic cigarette 200 is electrified, the heating component 212 generates heat, and the heat preheats air within the cigarette body 210. Because the temperature sensing end 221 of the temperature sensing element 220 is located within the air inlet passage 214, the heat generated by the heating component 212 radiates to the temperature sensing end 221 of the temperature sensing element 220. When inhaling is not performed, the temperature after preheating detected by the temperature sensing element 220 is a first temperature value. In this case, the temperature detected by the temperature sensing element 220 is higher than the temperature of the outside air.

When an inhaling action is generated, outside air enters the air inlet passage 214 from the air inlet 214a and flows into the accommodating cavity 211a, and an airflow flowing within the airflow passage is formed. Because the temperature sensing element 221 of the temperature sensing element 220 is located within the air inlet passage 214, is close to the air inlet 214a, and is also close to the heating component 212, in this case, a temperature value detected by the temperature sensing element 220 is a second temperature value, the second temperature value is equivalent to a temperature value of cold air from the outside, and the second temperature value is lower than the first temperature value. In addition, there is a relatively large temperature difference between the second temperature value and the first temperature value, and detection sensitivity can be improved. Therefore, when inhaling is performed and when inhaling is not performed, the temperature sensing end 221 detects different temperature values to obtain change of the temperature. The circuit board 230 obtains the number of times of inhaling according to the change of the temperature. Therefore, to obtain the number of times of inhaling, a negative pressure does not need to be sensed, but only the temperature sensing end 221 of the temperature sensor needs to detect the change of the temperature. Therefore, detection accuracy of the number of times of inhaling can be effectively improved.

Referring to FIG, 7, an electronic cigarette 300 in a fourth implementation includes a cigarette body 310, a temperature sensing element 320, and a circuit board 330. The cigarette body 310 and the temperature sensing element 320 are separately electrically connected to the circuit board 330.

Specifically, in the fourth implementation, the cigarette body 310 includes a cylinder 311, and the cylinder 311 and the circuit board 330 are electrically connected by using a conductive wire 312. The cylinder 311 has an accommodating cavity 311a, an air inlet passage 313 is located at one end of the cylinder 311, the air inlet passage 313 has an air inlet 314, and the air inlet passage 313 and the accommodating cavity 311a are in communication to form an airflow passage. That is, the air inlet passage 313 is located at the bottom of the cylinder 311.

The cylinder 311 is directly connected to the circuit board 330 by using the conductive wire 312. Therefore, after being electrified, the cylinder 311 generates heat, and the heat serves as a heat source to provide heat to a tobacco product. The tobacco product is directly placed within the accommodating cavity 311a of the cylinder 311, and heat conduction is directly performed on the tobacco product by using an inner wall of the cylinder 311, to heat the tobacco product.

The temperature sensing element 320 includes a temperature sensing end 321, the temperature sensing end 321 is disposed within the airflow passage, and the temperature sensing element 320 is electrically connected to the circuit board 330. Specifically, the temperature sensing end 321 of the temperature sensing element 320 is located within the air inlet passage 313. Therefore, the temperature sensing end 321 of the temperature sensing element 320 is relatively close to the air inlet 314 and is also relatively close to the cylinder 311. The temperature sensing element 320 may be a temperature sensor, a negative temperature coefficient (NTC) thermistor, or a thermocouple.

In the foregoing fourth implementation, after the electronic cigarette 300 is electrified, the cylinder 311 generates heat, and the heat preheats air within the cigarette body 310 and the tobacco product within the cylinder 311. Because the temperature sensing end 321 of the temperature sensing element 320 is located within the air inlet passage 313, the heat generated by the cylinder 311 radiates to the temperature sensing end 321 of the temperature sensing element 320. When inhaling is not performed, the temperature after preheating detected by the temperature sensing element 320 is a first temperature value. In this case, the temperature detected by the temperature sensing element 320 is higher than the temperature of the outside air.

When an inhaling action is generated, outside air enters the air inlet passage 313 from the air inlet 314 and flows into the accommodating cavity 311a, and an airflow flowing within the airflow passage is formed. Because the temperature sensing element 321 of the temperature sensing element 320 is located within the air inlet passage 313, is close to the air inlet 314, and is also close to the heating component, in this case, a temperature value detected by the temperature sensing element 320 is a second temperature value, the second temperature value is equivalent to a temperature value of cold air from the outside, and the second temperature value is lower than the first temperature value. In addition, there is a relatively large temperature difference between the second temperature value and the first temperature value, and detection sensitivity can be improved. Therefore, when inhaling is performed and when inhaling is not performed, the temperature sensing end 321 detects different temperature values to obtain change of the temperature. The circuit board 330 obtains the number of times of inhaling according to the change of the temperature. Therefore, to obtain the number of times of inhaling, a negative pressure does not need to be sensed, but only the temperature sensing end 321 of the temperature sensor needs to detect the change of the temperature. Therefore, detection accuracy of the number of times of inhaling can be effectively improved.

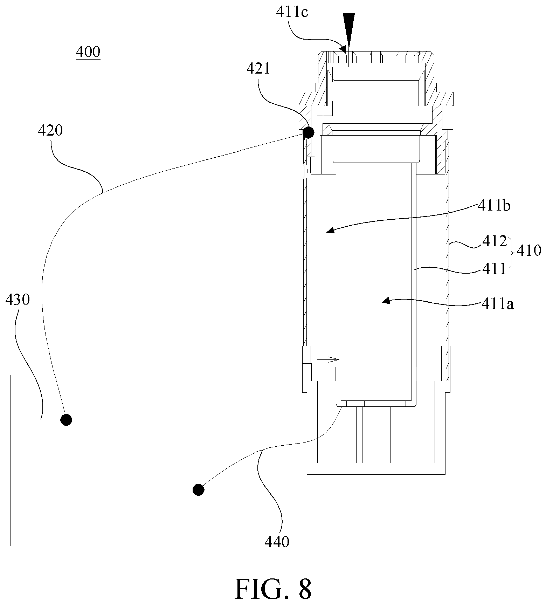

Referring to FIG. 8, an electronic cigarette 400 in a fifth implementation includes a cigarette body 410, a temperature sensing element 420, and a circuit board 430. The cigarette body 410 and the temperature sensing element 420 are separately electrically connected to the circuit board 430.

Specifically, in the fifth implementation, the cigarette body 410 includes a cylinder 411 and a sleeve 412. The sleeve 412 is disposed outside the cylinder 411, and the cylinder 411 is electrically connected to the circuit board 430 by using the conductive wire 440. The cylinder 411 has an accommodating cavity 411a, and the accommodating cavity 411a is used to place a tobacco product. After being electrified, cylinder 411 generates heat, and the heat heats air within the cigarette body 410.

The sleeve 412 is sleeved outside the cylinder 411, an air inlet passage 411b is formed between the sleeve 412 and the cylinder 411, the air inlet passage 411b has an air inlet 411c, and the air inlet passage 411b and the accommodating cavity 411a are in communication to form an airflow passage. During inhaling, outside cold air enters the air inlet passage 411b through the air inlet 411c under an inhaling force and enters the accommodating cavity 411a, and an airflow flowing within the airflow passage is formed (as shown in a dotted arrow direction in FIG. 8).

The temperature sensing element 420 includes a temperature sensing end 421, the temperature sensing end 421 is disposed within the airflow passage, and the temperature sensing element 420 is electrically connected to the circuit board 430. The temperature sensing element 420 may be a temperature sensor, a negative temperature coefficient (NTC) thermistor or a thermocouple. A hole is provided on the side wall of the sleeve 412. The temperature sensing end 421 of the temperature sensing element 420 extends into the airflow passage through the hole, and then the hole is sealed. The temperature sensing end 421 of the temperature sensing element 420 is located within the air inlet passage 411b and is close to the air inlet 411c.

In the foregoing fifth implementation, after the electronic cigarette 400 is electrified, the cylinder 411 generates heat, and the heat preheats air within the cigarette body 410 and the tobacco product placed within the cylinder 411. Because the temperature sensing end 421 of the temperature sensing element 420 is located within the air inlet passage 411b, the heat generated by the cylinder 411 radiates to the temperature sensing end 421 of the temperature sensing element 420. When inhaling is not performed, the temperature after preheating detected by the temperature sensing element 420 is a first temperature value. In this case, the temperature detected by the temperature sensing element 420 is higher than the temperature of the outside air.

When an inhaling action is generated, outside air enters the air inlet passage 411b from the air inlet 411c and flows into the accommodating cavity 411a, and an airflow flowing within the airflow passage is formed. Because the temperature sensing element 420 is close to the air inlet 411c, in this case, a temperature value detected by the temperature sensing element 420 is a second temperature value, the second temperature value is equivalent to a temperature value of cold air from the outside, and the second temperature value is lower than the first temperature value. Therefore, when inhaling is performed and when inhaling is not performed, the temperature sensing end 421 detects different temperature values to obtain change of the temperature. The circuit board 430 obtains the number of times of inhaling according to the change of the temperature. Therefore, to obtain the number of times of inhaling, a negative pressure does not need to be sensed, but only the temperature sensing end 421 of the temperature sensor needs to detect the change of the temperature. Therefore, detection accuracy of the number of times of inhaling can be effectively improved.

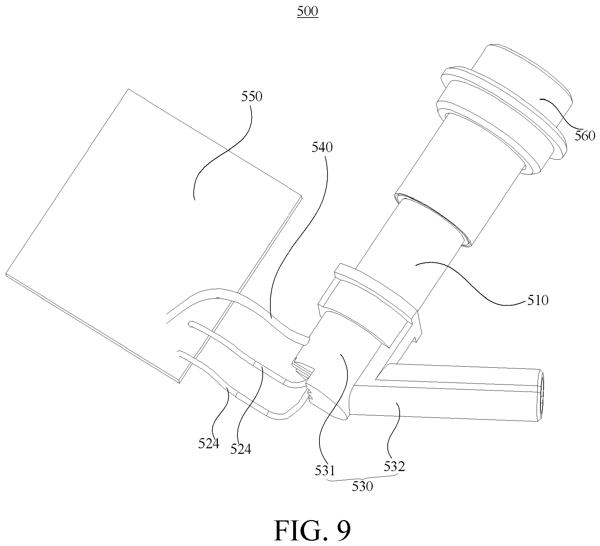

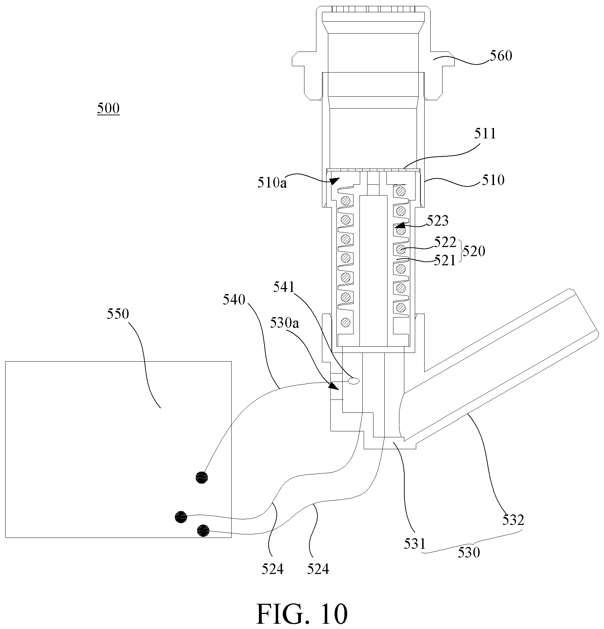

Referring to FIG. 9 and FIG. 10, an electronic cigarette 500 in a sixth implementation includes a cylinder 510, a heating component 520, an air inlet passage 530, a temperature sensing element 540, and a circuit board 550. The cylinder 510 has an accommodating cavity 510a, and the heating component 520 is accommodated within the accommodating cavity 510a.

Specifically, in the sixth implementation, the cylinder 510 is further provided with a filter screen 511. The filter screen 511 divides the cylinder 510 into an upper cavity and a lower cavity. The upper cavity and the lower cavity are in communication. The heating component 520 is located within the lower cavity, and the upper cavity is used to accommodate a tobacco product. The filter screen 511 is mainly used to filter scrapes and the like generated by the tobacco product disposed within the upper cavity, and prevent the scrapes from falling on the heating component 520 below the filter screen 511.

Specifically, in the sixth implementation, the heating component 520 includes a thermally conductive rod 521 and a heating wire 522. The heating wire 522 is winded on the thermally conductive rod 521. Two ends of the heating wire 522 are separately electrically connected to the circuit board 550. The cylinder 510 may be made of an insulating and easily thermally conductive material. In this case, the cylinder 510 mainly conducts heat generated by the heating wire 522. For example, the cylinder 510 may be an easily thermally conductive metal, ceramic, or another medium. The cylinder 510 may also be a thin aluminum material after insulation treatment or the like. The circuit board 550 may be a printed circuit board assembly (PCBA) board.

The material of the filter screen 511 may be an easily thermally conductive and easily cleaned material, for example, a metal or a stainless steel. By means of close fitting of the thermally conductive rod 521 and the filter screen 511, heat generated by the heating wire 522 can be fast conducted onto the filter screen 511 through the thermally conductive rod 521, and the filter screen 511 is in direct contact with the tobacco product. Therefore, filter screen 511 can heat the tobacco product, thereby improving heating efficiency.

A spiral groove 523 is provided on an outer side wall of the thermally conductive rod 521. The spiral groove 523 extends along an axial direction of the thermally conductive rod 521. The heating wire 522 is winded on the thermally conductive rod 521 in a spiral shape along the spiral groove 523. The heating wire 522, the outer side wall of the thermally conductive rod 521, a side wall of the spiral groove 523, and an inner side wall of the cylinder 510 together form a flow guide passage through encircling. The height of the side wall of the spiral groove 523 is greater than a diameter size of the heating wire 522, to ensure the size of the flow guide passage. Because the heating wire 522 is winded on the outer side wall of the thermally conductive rod 521 in a spiral shape along the spiral groove 523, an airflow is directly heated by using the heating wire 522, heat generated by the heating wire 522 may also make the temperature of the thermally conductive rod 521 rise. After the temperature of the thermally conductive rod 521 rises, the airflow may also be heated. The heating wire 522 is electrically connected to the circuit board by using a wire 524.

One end of the air inlet passage 530 is sleeved on one end of the cylinder 510, and the air inlet passage 530 and the accommodating cavity 510a are in communication to form an airflow passage. When an inhaling action is generated, outside cold air may enter the air inlet passage 530 under an inhaling force and enters the accommodating cavity 510a, and an airflow flows within the airflow passage.

The temperature sensing element 540 includes a temperature sensing end 541. The temperature sensing end 541 is disposed within the airflow passage and is located at an end portion close to the heating component 520. The temperature sensing end 541 is used to detect the temperature of the airflow within the airflow passage. The circuit board 550 is electrically connected to the heating component 520, and the circuit board 550 is electrically connected to the other end of the temperature sensing element 540.

Specifically, in the sixth implementation, a through hole 530a is provided on a side wall of the air inlet passage 530, and the temperature sensing end 541 of the temperature sensing element 540 passes through the through hole 530a and extends into the air inlet passage 530, and is located at the end portion of the heating component 520. Specifically, the temperature sensing element 540 may be a temperature sensor, a negative temperature coefficient (NTC) thermistor, or a thermocouple.

The air inlet passage 530 includes a first passage 531 and a second passage 532. The first passage 531 and the second passage 532 are disposed at a preset angle. For example, the first passage 531 and the second passage 532 are disposed at an acute angle. Certainly, in other implementations, the first passage 531 may also be disposed vertical to the second passage 532. The first passage 531 is sleeved at one end of the cylinder 510, and one end of the second passage 532 is disposed at the other end of the first passage 531 and is in communication with the first passage 531.

Specifically, the through hole 530a is provided on a side wall of the first passage 531, and the temperature sensing end 541 of the temperature sensing element 540 passes through the through hole 530a and extends into the first passage 531. Therefore, the temperature sensing end 541 is relatively close to the heating component 520. When inhaling is not performed, heat radiated by the heating component 520 to the temperature sensing end 541 is relatively large, and a first temperature value detected by the temperature sensing end 541 is relatively large. After the temperature sensing end 541 of the temperature sensing element 540 passes through the through hole 530a and extends into the first passage 531, the through hole 530a is sealed by using a sealant to prevent air leakage.

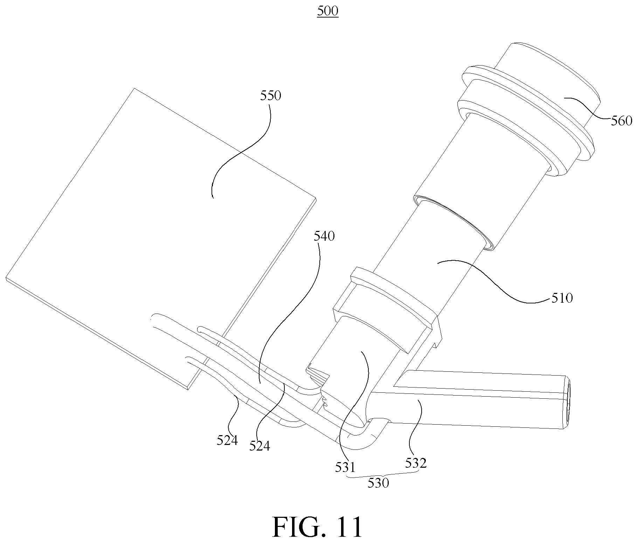

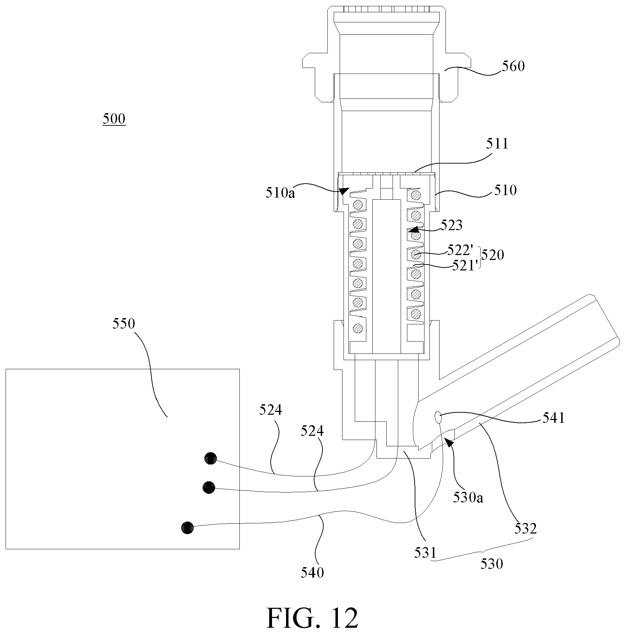

Certainly, referring to FIG. 11 and FIG. 12, in a seventh implementation, the through hole 530a is provided on a side wall of the second passage 532, and the temperature sensing end 541 of the temperature sensing element 540 passes through the through hole 530a and extends into the second passage 532. Therefore, a distance between the temperature sensing end 541 and the heating component 520 is longer than that in the sixth implementation, and the first temperature value detected by the temperature sensing end 541 is smaller than that in the sixth implementation.

In a seventh implementation, the heating component 520 includes a heating rod 521' and a thermally conductive wire 522'. The heating rod 521' is electrically connected to the circuit board 550 by using a wire 524, and the thermally conductive wire 522' is winded on the heating rod 521' in a spiral shape.

Specifically, in the sixth and seventh implementations, the electronic cigarette 500 further includes a thermal insulation base 560, and the thermal insulation base 560 is sleeved on the other end of the cylinder 510.

After the foregoing two electronic cigarettes 500 are electrified, the heating component 520 generates heat, and the heat heats air around the heating component 520. Because the temperature sensing end 541 of the temperature sensing element 540 extends into the air inlet passage 530 and is located at an end portion close to the heating component 520, heat generated after the heating component 520 is electrified radiates to the temperature sensing end 541 of the temperature sensing element 540. When inhaling starts, a temperature value detected by the temperature sensing end 541 is a first temperature value.

During inhaling, an inhaling force makes a flowing airflow be formed within the airflow passage, and outside cold air enters through the air inlet passage 530. When inhaling stops, the temperature of an airflow passing through the temperature sensing end 541 of the temperature sensing element 540 decreases, the temperature sensing end 541 detects that a decreased temperature value is a second temperature value. There is a temperature difference between the second temperature value and the first temperature value, The circuit board 550 receives the temperature difference and converts the temperature difference into an inductive signal. The number of times of inhaling is obtained by detecting the inductive signal. Therefore, to obtain the number of times of inhaling, a negative pressure does not need to be sensed, but only the temperature sensing end 541 of the temperature sensing element 540 needs to detect the change of the temperature. Therefore, detection accuracy of the number of times of inhaling can be effectively improved.

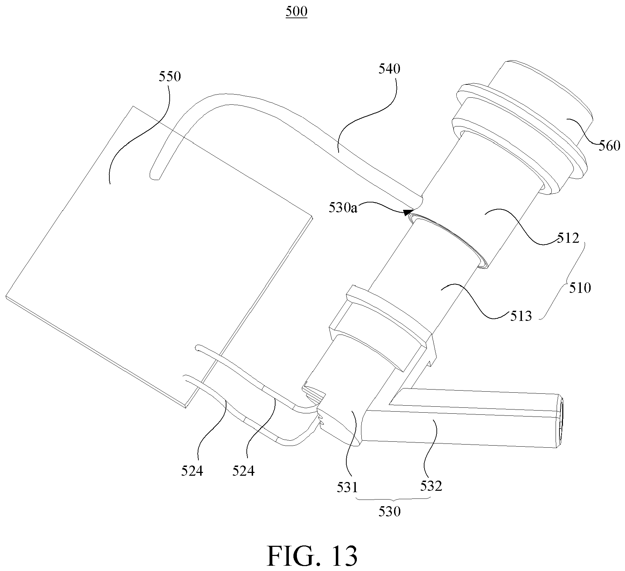

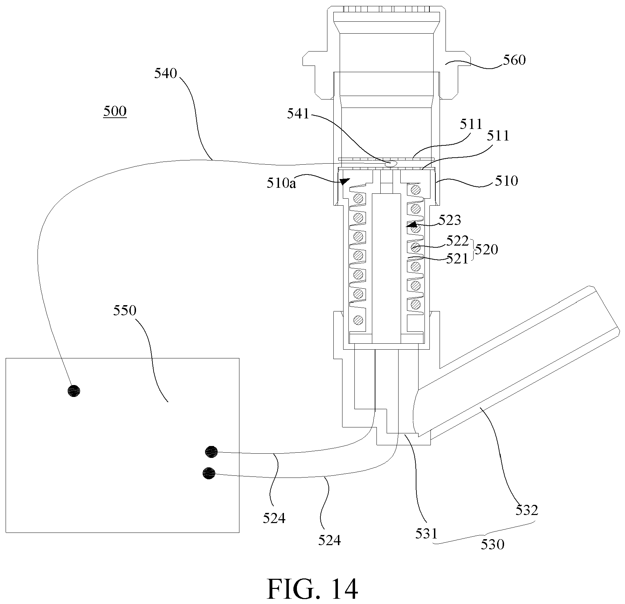

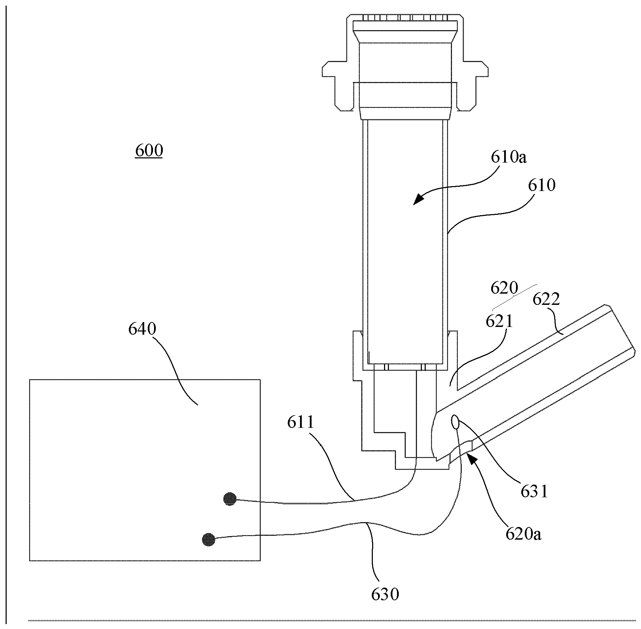

Referring to FIG. 13 and FIG. 14, in an electronic cigarette 500 in an eighth implementation, a cylinder 510 includes a main body 513 and a heating jacket 512, and an air inlet passage 530 and the heating jacket 512 are respectively sleeved on two ends of the main body 513. A through hole 530a is provided on a side wall of the heating jacket 512. After passing through the through hole 530a, a temperature sensing end 541 of a temperature sensing element 540 extends into the heating jacket 512 and is located at an end portion close to the heating component 520. That is, in the eighth implementation, the temperature sensing end 541 of the temperature sensing element 540 is located at an upper end of the heating component 520.

Specifically, in the eighth implementation, the electronic cigarette 500 further includes two filter screens 511, and the two filter screens 511 are accommodated within the cylinder 510. The two filter screens 511 are disposed at an interval, and the interval is relatively small. The temperature sensing end 541 of the temperature sensing element 540 is located between the two filter screens 511, to prevent the temperature sensing end 541 from directly contacting a tobacco product so as to affect detection accuracy. The two filter screens 511 are located above the heating component 520 and divides the cylinder 510 into an upper cavity and a lower cavity.

After the electronic cigarette 500 is electrified, the heating component 520 generates heat, and the heat heats air around the heating component 520. Because the temperature sensing end 541 of the temperature sensing element 540 extends into a heating jacket 512 and is located at an end portion close to the heating component 520, heat generated after the heating component 520 is electrified radiates to the temperature sensing end 541 of the temperature sensing element 540. When inhaling starts, a temperature value detected by the temperature sensing end 541 is a first temperature value.

During inhaling, an inhaling force makes a flowing airflow be formed in an airflow passage, and air heated by the heating component 520 flows upward. When inhaling stops, the temperature of an airflow passing through the temperature sensing end 541 of the temperature sensing element 540 decreases, the temperature sensing end 541 detects that a decreased temperature value is a second temperature value. There is a temperature difference between the second temperature value and the first temperature value. The circuit board 550 receives the temperature difference and converts the temperature difference into an inductive signal. The number of times of inhaling is obtained by detecting the inductive signal. Therefore, to obtain the number of times of inhaling, a negative pressure does not need to be sensed, but only the temperature sensing end 541 of the temperature sensing element 540 needs to detect the change of the temperature. Therefore, detection accuracy of the number of times of inhaling can be effectively improved.

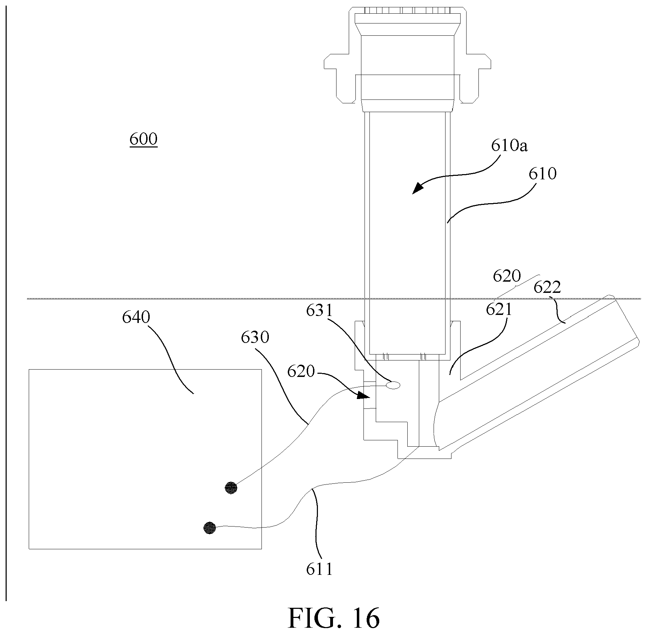

Referring to FIG. 16, an electronic cigarette 600 in a ninth implementation is heated in a conductive heating manner. Specifically, the electronic cigarette 600 includes a cylinder 610, an air inlet passage 620, a temperature sensing element 630, and a circuit board 640.

The cylinder 610 has an accommodating cavity 610a, and the accommodating cavity 610a is used to accommodate a tobacco product. After being electrified, the cylinder 610 generates heat, and the generated heat directly bakes and heats the tobacco product. Therefore, the cylinder 610 is a heating cylinder. For example, the cylinder 610 may be made of a high temperature resistant and easily thermally conductive material. The cylinder 610 is electrically connected to the circuit board 640 by using a wire 611.

One end of the air inlet passage 620 is sleeved on one end of the cylinder 610, and the air inlet passage 620 and the accommodating cavity 610a are in communication to form an airflow passage. A through hole 620a is provided on a side wall of the air inlet passage 620.

The temperature sensing element 630 includes a temperature sensing end 631. The temperature sensing end 631 passes through the through hole 620a and extends into the air inlet passage 620, and is located close to the cylinder 610. The circuit board 640 is electrically connected to the cylinder 610, and the circuit board 640 is electrically connected to the other end of the temperature sensing element 630.

Specifically, in the ninth implementation, the air inlet passage 620 includes a first passage 621 and a second passage 622. The first passage 621 and the second passage 622 are disposed at a preset angle. For example, the first passage 621 and the second passage 622 are disposed at an acute angle. Certainly, in other implementations, the first passage 621 may also be disposed vertical to the second passage 622. The first passage 621 is sleeved at one end of the cylinder 610, and one end of the second passage 622 is disposed at the other end of the first passage 621 and is in communication with the first passage 621. The through hole 620a is provided on a side wall of the first passage 621, and the temperature sensing end 631 of the temperature sensing element 630 passes through the through hole 620a and extends into the first passage 621. Therefore, the temperature sensing end 631 of the temperature sensing element 630 is relatively close to an end portion of the cylinder 610. Heat generated after the cylinder 610 is electrified radiates to the temperature sensing end 631.

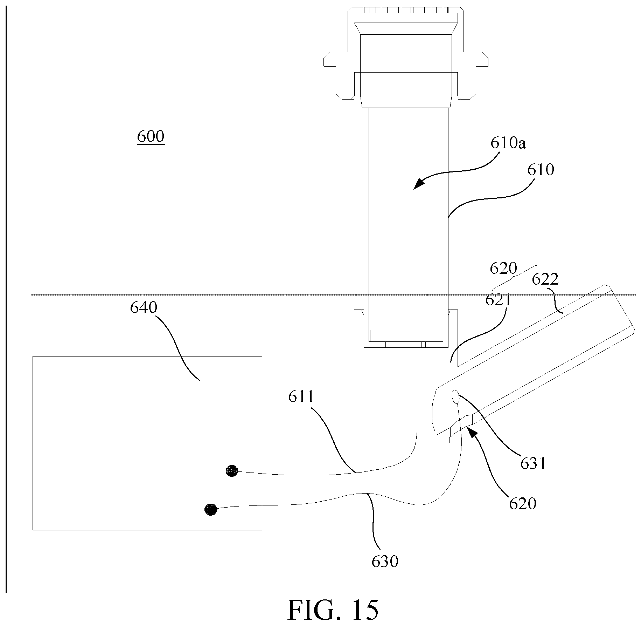

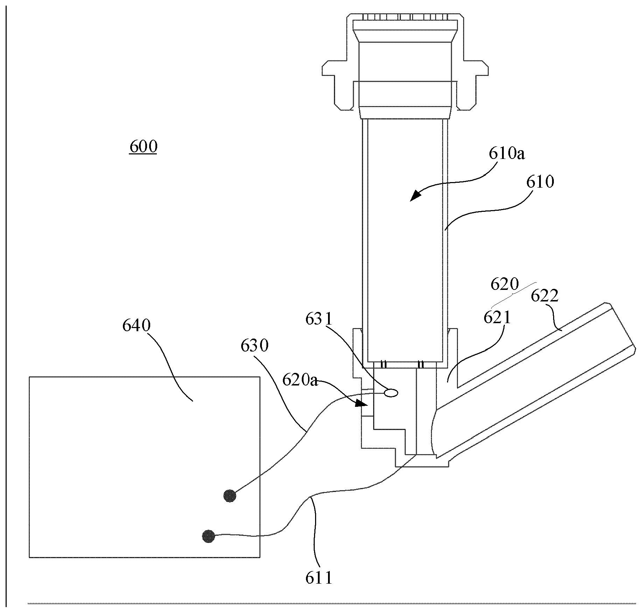

Certainly, referring to FIG. 15, in the electronic cigarette 600 in the tenth implementation, the air inlet passage 620 includes a first passage 621 and a second passage 622. The first passage 621 and the second passage 622 are disposed at a preset angle. For example, the first passage 621 and the second passage 622 are disposed at an acute angle. Certainly, in other implementations, the first passage 621 may also be disposed vertical to the second passage 622. The first passage 621 is sleeved at one end of the cylinder 610, and one end of the second passage 622 is disposed at the other end of the first passage 621 and is in communication with the first passage 621. The through hole 620a is provided on a side wall of the second passage 622, and the temperature sensing end 631 of the temperature sensing element 630 passes through the through hole 620a and extends into the second passage 622. Therefore, a distance between the temperature sensing end 631 of the temperature sensing element 630 and the cylinder 610 is longer than that in the ninth implementation.

After the electronic cigarette 600 is electrified, the cylinder 610 generates heat, and the heat is directly conducted to a tobacco product. Because the temperature sensing end 631 of the temperature sensing element 630 extends into the air inlet passage 620 and is located at an end portion close to the cylinder 610, heat generated after the cylinder 610 is electrified radiates to the temperature sensing end 631 of the temperature sensing element 630. When inhaling starts, a temperature value detected by the temperature sensing end 631 is a first temperature value.

During inhaling, an inhaling force makes a flowing airflow be formed within the airflow passage, and cold air enters from the air inlet passage 620. When inhaling stops, the temperature of an airflow passing through the temperature sensing end 631 of the temperature sensing element 630 decreases, the temperature sensing end 631 detects that a decreased temperature value is a second temperature value. There is a temperature difference between the second temperature value and the first temperature value. The circuit board 640 receives the temperature difference and converts the temperature difference into an inductive signal. The number of times of inhaling is obtained by detecting the inductive signal. Therefore, to obtain the number of times of inhaling, a negative pressure does not need to be sensed, but only the temperature sensing end 631 of the temperature sensing element 630 needs to detect the change of the temperature. Therefore, detection accuracy of the number of times of inhaling can be effectively improved.

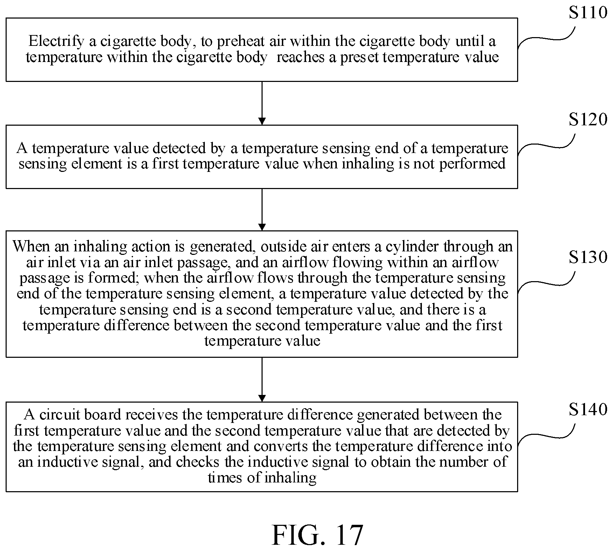

Referring to FIG. 17, a method for detecting the number of times of inhaling of an electronic cigarette 100 is further provided and specifically includes the following steps:
Step S110: Electrify a cigarette body 110, to preheat air within the cigarette body 110 until a temperature within the cigarette body 110 reaches a preset temperature value.

The preset temperature value may be a reasonable temperature range value and is not limited to one or more point values. In an implementation, the electrifying a cigarette body 110 is specifically electrifying a heating component 112. After being electrified, the heating component 112 generates heat, and the heat preheats air around the heating component 112. In another implementation, the electrifying a cigarette body 110 is specifically electrifying a cylinder 310. The cylinder 310 is used as a heating cylinder. After being electrified, the heating cylinder directly conductively heats a tobacco product accommodated in an accommodating cavity.

Step S120: A temperature value detected by a temperature sensing end of a temperature sensing element is a first temperature value when inhaling is not performed.

Step S130: When an inhaling action is generated, outside air enters a cylinder 111 through an air inlet 111c via an air inlet passage 111b, and an airflow flowing within an airflow passage is formed; when the airflow flows through the temperature sensing end of the temperature sensing element, a temperature value detected by the temperature sensing end is a second temperature value, and there is a temperature difference between the second temperature value and the first temperature value.

Specifically, the first temperature value may be higher than the second temperature value. In this case, the temperature sensing end 121 of the temperature sensing element 120 is located at a position close to an air source. Certainly, in other implementations, the first temperature value may be lower than the second temperature value. In this case, the temperature sensing end 121 of the temperature sensing element 120 is located at a position close to a heat source.

Step S140: Receiving the temperature difference generated between the first temperature value and the second temperature value that are detected by the temperature sensing element 120 and converting the temperature difference into an inductive signal, and checking the inductive signal to obtain the number of times of inhaling by a circuit board 130. The process is repeated in this way. Different temperature values are detected when inhaling is not performed and when inhaling is performed, thereby determining the number of times of inhaling.

In the foregoing method for detecting the number of times of inhaling of the electronic cigarette 100, the cigarette body 110 is electrified, to preheat air within the cigarette body 110 until the temperature within the cigarette body 110 reaches the preset temperature value. The temperature value detected by the temperature sensing end 121 of the temperature sensing element 120 is the first temperature value when inhaling is not performed. When the inhaling action is generated, outside air enters a cylinder 111 through the air inlet 111c via the air inlet passage 111b, and the airflow flowing within the airflow passage 111b is formed; when the airflow flows through the temperature sensing end 121 of the temperature sensing element 120, the temperature value detected by the temperature sensing end 121 is the second temperature value, and there is a temperature difference between the second temperature value and the first temperature value. The circuit board 130 receives the temperature difference generated between the first temperature value and the second temperature value that are detected by the temperature sensing element 120 and converts the temperature difference into the inductive signal, and detects the inductive signal to obtain the number of times of inhaling. Therefore, to obtain the number of times of inhaling, a negative pressure does not need to be sensed, but only the temperature sensing end of the temperature sensor needs to detect the change of the temperature. Therefore, detection accuracy of the number of times of inhaling can be effectively improved.

## Claims

1. An electronic cigarette (100, 200, 300, 400, 500, 600), **characterized by** comprising:
a cigarette body (110, 210, 310, 410), wherein the cigarette body (110, 210, 310, 410) comprises a cylinder (111, 211, 311, 411, 510, 610), the cylinder (111, 211, 311, 411, 510, 610) has an accommodating cavity (111a, 211a, 311a, 411a, 510a, 610a), the cigarette body (110, 210, 310, 410) is further provided with an air inlet passage (111b, 214, 313, 411b, 530, 620), the air inlet passage (111b, 214, 313, 411b, 530, 620) has an air inlet (111c, 214a, 314, 411c), and the air inlet passage (111b, 214, 313, 411b, 530, 620) is in communication with the accommodating cavity 111a, 211a, 311a, 411a, 510a, 610a) to form an airflow passage;
a temperature sensing element (120, 220, 320, 420, 540, 630), wherein the temperature sensing element (120, 220, 320, 420, 540, 630) comprises a temperature sensing end (121, 221, 321, 421, 541, 631), the temperature sensing end (121, 221, 321, 421, 541, 631) is disposed within the airflow passage, when an inhaling action is generated, outside air enters the air inlet passage (111b, 214, 313, 411b, 530, 620) from the air inlet (111c, 214a, 314, 411c) and flows into the accommodating cavity (111a, 211a, 311a, 411a, 510a, 610a), an airflow flowing within the airflow passage is formed, the temperature sensing end (121, 221, 321, 421, 541, 631) of the temperature sensing element (120, 220, 320, 420, 540, 630) is configured to detect a temperature of the airflow when inhaling is performed and when inhaling is not performed to learn of change of the temperature and obtain the number of times of inhaling according to the change of the temperature; and
a circuit board (130, 230, 330, 430, 550, 640), wherein the circuit board (130, 230, 330, 430, 550, 640) is electrically connected to the temperature sensing element (120, 220, 320, 420, 540, 630), and the circuit board (130, 230, 330, 430, 550, 640) is electrically connected to the cigarette body (110, 210, 310, 410) ;
the circuit board (130, 230, 330, 430, 550, 640) receiving the temperature difference generated between the first temperature value and the second temperature value that are detected by the temperature sensing element (120, 220, 320, 420, 540, 630) and converting the temperature difference into an inductive signal, and detecting the inductive signal to obtain the number of times of inhaling.

2. The electronic cigarette according to claim 1, **characterized in that** the cigarette body (110) further comprises a heating component (112), a filter screen (113), and a sleeve (114), the sleeve (114) is sleeved outside the cylinder (111), the air inlet passage (111b) is formed between an inner wall of the sleeve (114) and an outer wall of the cylinder (111), the cylinder (111) comprises a first cylinder (1111) and a second cylinder (1112), the filter screen (113) is located between the first cylinder (1111) and the second cylinder (1112), the heating component (112) is located within the first cylinder (1111), the second cylinder (1112) is used to place a tobacco product, and the heating component (112) is electrically connected to the circuit board (130).

3. The electronic cigarette according to claim 2, **characterized in that** the temperature sensing end (121) is located within the air inlet passage (111b) and is close to the air inlet (111c) ; or the temperature sensing end (121) is located within the cylinder (111) and is located at a position, close to the heating component (112), within the second cylinder (1112).

4. The electronic cigarette according to claim 1, **characterized in that** the cigarette body (210) further comprises a heating component (212) and a casing (213), the heating component (212) is accommodated within the accommodating cavity (211a) of the cylinder (211), the heating component (212) is electrically connected to the circuit board (230), the casing (213) is sleeved outside the cylinder (211), and the air inlet passage (214) is located at one end, provided with the heating component (212), of the cylinder (211).

5. The electronic cigarette according to claim 4, **characterized in that** the temperature sensing end (221) is located within the air inlet passage (214).

6. The electronic cigarette according to claim 1, **characterized in that** the cylinder (311) is electrically connected to the circuit board (330), the air inlet passage (313) is located at one end of the cylinder (311), and the temperature sensing end (321) of the temperature sensing element (320) is located within the air inlet passage (313) ; or the cigarette body (410) further comprises a sleeve (412), the sleeve (412) is disposed outside the cylinder (411), the cylinder (411) is electrically connected to the circuit board (430), the air inlet passage (411b) is formed between the sleeve (412) and the cylinder (411), and the temperature sensing end (421) of the temperature sensing element (420) is located within the air inlet passage (411b) and is close to the air inlet (411c).

7. The electronic cigarette according to claim 1, **characterized in that** the cigarette body further comprises a heating component (520), the heating component (520) is accommodated within the accommodating cavity (510a), one end of the air inlet passage (530) is sleeved on one end of the cylinder (510), and the temperature sensing end (541) is located at one end, close to the heating component (520), within the air inlet passage (530).

8. The electronic cigarette according to claim 7, **characterized in that** a through hole (530a) is provided on a side wall of the air inlet passage (530), and the temperature sensing end (541) passes through the through hole (530a) and extends into the air inlet passage (530), and is located at an end portion close to the heating component (520).

9. The electronic cigarette according to claim 8, **characterized in that** the air inlet passage (530) comprises a first passage (531) and a second passage (532), the first passage (531) and the second passage (532) are disposed at a preset angle, the first passage (531) is sleeved at one end of the cylinder (510), one end of the second passage (532) is disposed at the other end of the first passage (531) and is in communication with the first passage (531), and the through hole (530a) is provided on a side wall of the first passage (531) ; or
the air inlet passage (530) comprises a first passage (531) and a second passage (532), the first passage (531) and the second passage (532) are disposed at a preset angle, the first passage (531) is sleeved at one end of the cylinder (510), one end of the second passage (532) is disposed at the other end of the first passage (531) and is in communication with the first passage (531), and the through hole (530a) is provided on a side wall of the second passage (532).

10. The electronic cigarette according to claim 7, **characterized in that** the cylinder (510) further includes a main body (513) and a heating jacket (512), and the air inlet passage (530) and the heating jacket (512) are respectively sleeved on two ends of the main body (513), a through hole (530a) is provided on a side wall of the heating jacket (512)., the temperature sensing end (541) of the temperature sensing element (540) passes through the through hole (530a) and extends into the heating jacket (512) and is located at an end portion close to the heating component (520).

11. The electronic cigarette according to claim 10, **characterized by** further comprising two filter screens (511), wherein the two filter screens (511) are accommodated within the cylinder (510), and the temperature sensing end (541) is located between the two filter screens (511).

12. The electronic cigarette according to claim 1, **characterized in that** the cylinder (610) is a heating cylinder, one end of the air inlet passage (620) is sleeved at one end of the heating cylinder, a through hole (620a) is provided on a side wall of the air inlet passage (620), and the temperature sensing end (631) passes through the through hole (620a) and extends into the air inlet passage (620), and is located at a position close to the heating cylinder.

13. The electronic cigarette according to claim 12, **characterized in that** the air inlet passage (620) comprises a first passage (621) and a second passage (622), the first passage (621) and the second passage (622) are disposed at a preset angle, the first passage (621) is sleeved at one end of the cylinder (610), one end of the second passage (622) is disposed at the other end of the first passage (621) and is in communication with the first passage (621), and the through hole (620a) is provided on a side wall of the first passage (621) ; or
the air inlet passage (620) comprises a first passage (621) and a second passage (622), the first passage (621) and the second passage (622) are disposed at a preset angle, the first passage (621) is sleeved at one end of the cylinder (610), one end of the second passage (622) is disposed at the other end of the first passage (621) and is in communication with the first passage (621), and the through hole (620a) is provided on a side wall of the second passage.

14. A method for detecting the number of times of inhaling of an electronic cigarette, **characterized by** comprising:
electrifying a cigarette body, to preheat air within the cigarette body until a temperature within the cigarette body reaches a preset temperature value, wherein
a temperature value detected by a temperature sensing end of a temperature sensing element is a first temperature value when inhaling is not performed;
when an inhaling action is generated, outside air enters a cylinder through an air inlet via an air inlet passage, and an airflow flowing within an airflow passage is formed; when the airflow flows through the temperature sensing end of the temperature sensing element, a temperature value detected by the temperature sensing end is a second temperature value, and there is a temperature difference between the second temperature value and the first temperature value; and
a circuit board receiving the temperature difference generated between the first temperature value and the second temperature value that are detected by the temperature sensing element and converting the temperature difference into an inductive signal, and detecting the inductive signal to obtain the number of times of inhaling.

15. The method according to claim 14, **characterized in that** the first temperature value is lower than the second temperature value; or
the first temperature value is higher than the second temperature value.

16. The method according to claim 14, **characterized in that** the electrifying a cigarette body, to preheat air within the cigarette body until a temperature within the cigarette body reaches a preset temperature value comprises: electrifying a heating component, wherein heat is generated after the heating component is electrified, and the heat preheats air around the heating component; or the electrifying a cigarette body, to preheat air within the cigarette body until a temperature within the cigarette body reaches a preset temperature value comprises: electrifying the cylinder, wherein the cylinder directly conductively heats a tobacco product accommodated within an accommodating cavity after being electrified.

## Patentansprüche

1. Elektronische Zigarette (100, 200, 300, 400, 500, 600), umfassend:
- einen Zigarettenkörper (110, 210, 310, 410), der einen Zylinder (111, 211, 311, 411, 510, 610) umfasst, welcher Zylinder (111, 211, 311, 411, 510, 610) eine Aufnahmekammer (111a, 211a, 311a, 411a, 510a, 610a) aufweist, wobei der Zigarettenkörper (110, 210, 310, 410) ferner mit einem Lufteinlasskanal (111b, 214, 313, 411b, 530, 620) versehen ist, der eine Lufteinlassöffnung (111c, 214a, 314, 411c) aufweist und mit der Aufnahmekammer (111a, 211a, 311a, 411a, 510a, 610a) verbunden ist, um einen Luftstromkanal zu erzeugen,
- ein Temperaturerfassungselement (120, 220, 320, 420, 540, 630), das ein Temperaturerfassungsende (121, 221, 321, 421, 541, 631) umfasst, welches Temperaturerfassungsende (121, 221, 321, 421, 541, 631) innerhalb des Luftstromkanals angeordnet ist, wobei beim Ziehen an der Zigarette die Außenluft über die Lufteinlassöffnung (111c, 214a, 314, 411c) in den Lufteinlasskanal (111b, 214, 313, 411b, 530, 620) eintritt und in die Aufnahmekammer (111a, 211a, 311a, 411a, 510a, 610a) einströmt, so dass ein innerhalb des Luftstromkanals fließender Luftstrom entsteht, wobei das Temperaturerfassungsende (121, 221, 321, 421, 541, 631) des Temperaturerfassungselements (120, 220, 320, 420, 540, 630) dazu ausgebildet ist, Temperaturen des Luftstroms beim Ziehen und Nicht-Ziehen an der Zigarette zu erfassen, um dadurch eine Temperaturänderung und in Abhängigkeit von der Temperaturänderung die Anzahl der gemachten Züge zu ermitteln, und
- eine Platine (130, 230, 330, 430, 550, 640), die sowohl mit dem Temperaturerfassungselement (120, 220, 320, 420, 540, 630) als auch mit dem Zigarettenkörper (110, 210, 310, 410) elektrisch verbunden ist, wobei die Platine (130, 230, 330, 430, 550, 640) eine Temperaturdifferenz zwischen einem ersten Temperaturwert und einem zweiten Temperaturwert, welche von dem Temperaturerfassungselement (120, 220, 320, 420, 540, 630) erfasst wurden, empfängt, diese Temperaturdifferenz in ein induktives Signal umwandelt und durch Erfassen des induktiven Signals die Anzahl der gemachten Züge ermittelt.

2. Elektronische Zigarette nach Anspruch 1, **dadurch gekennzeichnet, dass** der Zigarettenkörper (110) ferner eine Heizbaugruppe (112), ein Filtersieb (113) und eine Hülse (114) umfasst, welche Hülse (114) den Zylinder (111) von außen so umhüllt, dass zwischen der Innenwand der Hülse (114) und der Außenwand des Zylinders (111) ein Lufteinlasskanal (111b) entsteht, wobei der Zylinder (111) einen ersten Zylinder (1111) und einen zweiten Zylinder (1112) umfasst, zwischen welchem ersten Zylinder (1111) und welchem zweiten Zylinder (1112) sich das Filtersieb (113) befindet, wobei die Heizbaugruppe (112) innerhalb des ersten Zylinders (1111) angeordnet und mit der Platine (130) elektrisch verbunden ist, während der zweite Zylinder (1112) zur Ablagerung eines Tabakprodukts dient.

3. Elektronische Zigarette nach Anspruch 2, **dadurch gekennzeichnet, dass** das Temperaturerfassungsende (121) innerhalb des Lufteinlasskanals (111b) in der Nähe der Lufteinlassöffnung (111c) angeordnet ist, oder dass das Temperaturerfassungsende (121) innerhalb des Zylinders (111) an einer nahe an der Heizbaugruppe (112) liegenden Stelle des zweiten Zylinders (1112) angeordnet ist.

4. Elektronische Zigarette nach Anspruch 1, **dadurch gekennzeichnet, dass** der Zigarettenkörper (210) ferner eine Heizbaugruppe (212) und eine Buchse (213) umfasst, wobei die Heizbaugruppe (212) innerhalb der Aufnahmekammer (211a) des Zylinders (211) aufgenommen und mit der Platine (230) elektrisch verbunden ist, während die Buchse (213) den Zylinder (211) von außen umhüllt und der Lufteinlasskanal (214) an dem mit der Heizbaugruppe (212) versehenen Ende des Zylinders (211) angeordnet ist.

5. Elektronische Zigarette nach Anspruch 4, **dadurch gekennzeichnet, dass** sich das Temperaturerfassungsende (221) innerhalb des Lufteinlasskanals (214) befindet.

6. Elektronische Zigarette nach Anspruch 1, **dadurch gekennzeichnet, dass** der Zylinder (311) mit der Platine (330) elektrisch verbunden ist, während sich der Lufteinlasskanal (313) an einem Ende des Zylinders (311) und das Temperaturerfassungsende (321) des Temperaturerfassungselements (320) innerhalb des Lufteinlasskanals (313) befindet, oder dass der Zigarettenkörper (410) ferner eine Hülse (412) umfasst, die außerhalb des mit der Platine (430) elektrisch verbundenen Zylinders (411) so angeordnet ist, dass zwischen der Hülse (412) und dem Zylinder (411) der Lufteinlasskanal (411b) entsteht, wobei das Temperaturerfassungsende (421) des Temperaturerfassungselements (420) innerhalb des Lufteinlasskanals (411b) in der Nähe der Lufteinlassöffnung (411c) angeordnet ist.

7. Elektronische Zigarette nach Anspruch 1, **dadurch gekennzeichnet, dass** der Zigarettenkörper ferner eine Heizbaugruppe (520) umfasst, die innerhalb der Aufnahmekammer (510a) aufgenommen ist, wobei ein Ende des Lufteinlasskanals (530) auf einem Ende des Zylinders (510) aufgesetzt ist und das Temperaturerfassungsende (541) an einem nahe an der Heizbaugruppe (520) liegenden Ende innerhalb des Lufteinlasskanals (530) angeordnet ist.

8. Elektronische Zigarette nach Anspruch 7, **dadurch gekennzeichnet, dass** in der Seitenwand des Lufteinlasskanals (530) ein Durchgangsloch (530a) ausgebildet ist, wobei das Temperaturerfassungsende (541) durch das Durchgangsloch (530a) hindurch in den Lufteinlasskanal (530) hineinragt und in einem nahe an der Heizbaugruppe (520) liegenden Endbereich angeordnet ist.

9. Elektronische Zigarette nach Anspruch 8, **dadurch gekennzeichnet, dass** der Lufteinlasskanal (530) einen ersten Kanal (531) und einen zweiten Kanal (532) umfasst, welcher erster Kanal (531) und welcher zweiter Kanal (532) in einem voreingestellten Winkel zueinander angeordnet sind, wobei der erste Kanal (531) auf einem Ende des Zylinders (510) aufgesetzt ist, während ein Ende des zweiten Kanals (532) an dem anderen Ende des ersten Kanals (531) angeordnet und mit dem ersten Kanal (531) verbunden ist, wobei das Durchgangsloch (530a) in der Seitenwand des ersten Kanals (531) ausgebildet ist, oder dass der Lufteinlasskanal (530) einen ersten Kanal (531) und einen zweiten Kanal (532) umfasst, welcher erster Kanal (531) und welcher zweiter Kanal (532) in einem voreingestellten Winkel zueinander angeordnet sind, wobei der erste Kanal (531) auf einem Ende des Zylinders (510) aufgesetzt ist, während ein Ende des zweiten Kanals (532) an dem anderen Ende des ersten Kanals (531) angeordnet und mit dem ersten Kanal (531) verbunden ist, wobei das Durchgangsloch (530a) in der Seitenwand des zweiten Kanals (532) ausgebildet ist.

10. Elektronische Zigarette nach Anspruch 7, **dadurch gekennzeichnet, dass** der Zylinder (510) ferner einen Hauptkörper (513) und einen Heizmantel (512) umfasst, wobei der Lufteinlasskanal (530) und der Heizmantel (512) auf beiden Enden des Hauptkörpers (513) aufgesetzt sind und das Durchgangsloch (530a) in der Seitenwand des Heizmantels (512) ausgebildet ist, während das Temperaturerfassungsende (541) des Temperaturerfassungselements (540) durch das Durchgangsloch (530a) hindurch in den Heizmantel (512) hineinragt und in einem nahe an der Heizbaugruppe (520) liegenden Endbereich angeordnet ist.

11. Elektronische Zigarette nach Anspruch 10, **dadurch gekennzeichnet, dass** sie ferner zwei Filtersiebe (511) umfasst, die innerhalb des Zylinders (510) aufgenommen sind, wobei sich das Temperaturerfassungsende (541) zwischen den beiden Filtersieben (511) befindet.

12. Elektronische Zigarette nach Anspruch 1, **dadurch gekennzeichnet, dass** der Zylinder (610) ein Heizzylinder ist, auf dessen einem Ende ein Ende des Lufteinlasskanals (620) aufgesetzt ist, wobei in der Seitenwand des Lufteinlasskanals (620) ein Durchgangsloch (620a) ausgebildet ist, wobei das Temperaturerfassungsende (631) durch das Durchgangsloch (620a) hindurch in den Lufteinlasskanal (620) hineinragt und in der Nähe des Heizzylinders angeordnet ist.

13. Elektronische Zigarette nach Anspruch 12, **dadurch gekennzeichnet, dass** der Lufteinlasskanal (620) einen ersten Kanal (621) und einen zweiten Kanal (622) umfasst, welcher erster Kanal (621) und welcher zweiter Kanal (622) in einem voreingestellten Winkel zueinander angeordnet sind, wobei der erste Kanal (621) auf einem Ende des Zylinders (610) aufgesetzt ist, während ein Ende des zweiten Kanals (622) an dem anderen Ende des ersten Kanals (621) angeordnet und mit dem ersten Kanal (621) verbunden ist, wobei das Durchgangsloch (620a) in der Seitenwand des ersten Kanals (621) ausgebildet ist, oder dass der Lufteinlasskanal (620) einen ersten Kanal (621) und einen zweiten Kanal (622) umfasst, welcher erster Kanal (621) und welcher zweiter Kanal (622) in einem voreingestellten Winkel zueinander angeordnet sind, wobei der erste Kanal (621) auf einem Ende des Zylinders (610) aufgesetzt ist, während ein Ende des zweiten Kanals (622) an dem anderen Ende des ersten Kanals (621) angeordnet und mit dem ersten Kanal (621) verbunden ist, wobei das Durchgangsloch (620a) in der Seitenwand des zweiten Kanals (622) ausgebildet ist.

14. Verfahren zum Erfassen die Anzahl an einer elektronischen Zigarette gemachter Züge, umfassend Folgendes:
- Bestromen eines Zigarettenkörpers zum Vorwärmen der innerhalb des Zigarettenkörpers vorhandenen Luft, bis die Temperatur innerhalb des Zigarettenkörpers einen voreingestellten Temperaturwert erreicht,
- bei Nicht-Ziehen an der Zigarette von einem Temperaturerfassungsende eines Temperaturerfassungselements ein erster Temperaturwert erfasst wird,
- beim Ziehen an der Zigarette die Außenluft über eine Lufteinlassöffnung durch einen Lufteinlasskanal in einen Zylinder eintritt, so dass ein innerhalb des Luftstromkanals fließender Luftstrom entsteht, wobei beim Vorbeiströmen des Luftstroms an dem Temperaturerfassungsende des Temperaturerfassungselements von dem Temperaturerfassungsende ein zweiter Temperaturwert erfasst wird, wobei zwischen dem zweiten Temperaturwert und dem ersten Temperaturwert eine Temperaturdifferenz besteht, und
- durch eine Platine die Temperaturdifferenz zwischen dem ersten Temperaturwert und dem zweiten Temperaturwert, welche von dem Temperaturerfassungselement erfasst wurden, empfangen, diese Temperaturdifferenz in ein induktives Signal umgewandelt und durch Erfassen des induktiven Signals die Anzahl der gemachten Züge ermittelt wird.

15. Verfahren nach Anspruch 14, **dadurch gekennzeichnet, dass** der erste Temperaturwert niedriger als der zweite Temperaturwert ist, oder dass der erste Temperaturwert höher als der zweite Temperaturwert ist.

16. Verfahren nach Anspruch 14, **dadurch gekennzeichnet, dass** das Bestromen des Zigarettenkörpers zum Vorwärmen der innerhalb des Zigarettenkörpers vorhandenen Luft, bis die Temperatur innerhalb des Zigarettenkörpers einen voreingestellten Temperaturwert erreicht, das Bestromen einer Heizbaugruppe umfasst, die im bestromten Zustand Wärme erzeugt, mit der die die Heizbaugruppe umgebende Luft vorgewärmt wird, oder dass das Bestromen des Zigarettenkörpers zum Vorwärmen der innerhalb des Zigarettenkörpers vorhandenen Luft, bis die Temperatur innerhalb des Zigarettenkörpers einen voreingestellten Temperaturwert erreicht, das Bestromen eines Zylinders umfasst, der im bestromten Zustand ein in einer Aufnahmekammer abgelagertes Tabakprodukt durch Wärmeleitung unmittelbar erwärmt.

## Revendications

1. Une cigarette électronique (100, 200, 300, 400, 500, 600) comprenant :
un corps de cigarette (110, 210, 310, 410), ledit corps de cigarette (110, 210, 310, 410) comprenant un cylindre (111, 211, 311, 411, 510, 610), ledit cylindre (111, 211, 311, 411, 510, 610) étant doté d'une cavité contenante (111a, 211a, 311a, 411a, 510a, 610a), ledit corps de cigarette (110, 210, 310, 410) étant en outre doté d'un conduit d'admission d'air (111b, 214, 313, 411b, 530, 620), ledit conduit d'admission d'air (111b, 214, 313, 411b, 530, 620) étant doté d'une ouverture d'admission d'air (111c, 214a, 314, 411c), ledit conduit d'admission d'air (111b, 214, 313, 411b, 530, 620) communiquant avec ladite cavité contenante (111a, 211a, 311a, 411a, 510a, 610a) pour former un conduit de flux d'air ;
un élément capteur de température (120, 220, 320, 420, 540, 630), ledit élément capteur de température (120, 220, 320, 420, 540, 630) comprenant une extrémité de captage de température (121, 221, 321, 421, 541, 631), ladite extrémité de captage de température (121, 221, 321, 421, 541, 631) étant agencée à l'intérieur dudit conduit de flux d'air, lorsqu'une action d'aspiration se produit, l'air externe pénètre ledit conduit d'admission d'air (111b, 214, 313, 411b, 530, 620) via ladite ouverture d'admission d'air (111c, 214a, 314, 411c), et s'écoule dans ladite cavité contenante (111a, 211a, 311a, 411a, 510a, 610a), formant un flux d'air s'écoulant à l'intérieur dudit conduit de flux d'air, ladite extrémité de captage de température (121, 221, 321, 421, 541, 631) dudit élément capteur de température (120, 220, 320, 420, 540, 630) est configurée de manière à détecter la température dudit flux d'air lors de l'aspiration ou de non aspiration afin d'obtenir les variations de température, et d'obtenir le nombre d'aspirations en fonction des variations de température ; et
un circuit imprimé (130, 230, 330, 430, 550, 640), ledit circuit imprimé (130, 230, 330, 430, 550, 640) et ledit élément capteur de température (120, 220, 320, 420, 540, 630) étant connectés électriquement, en outre ledit circuit imprimé (130, 230, 330, 430, 550, 640) et ledit corps de cigarette (110, 210, 310, 410) sont connectés électriquement, ledit circuit imprimé (130, 230, 330, 430, 550, 640) reçoit l'écart de température produit entre la première valeur de température et la deuxième valeur de température détectées par ledit élément capteur de température (120, 220, 320, 420, 540, 630), le convertit en signal inducteur et obtient le nombre d'aspirations en détectant le signal inducteur.

2. Une cigarette électronique selon la revendication 1, **caractérisée en ce que**, ledit corps de cigarette (110) comprend en outre un composant chauffant (112), un tamis de filtrage (113), et un manchon (114), ledit manchon (114) est emmanché sur l'extérieur dudit cylindre (111), la paroi interne dudit manchon (114) et la paroi externe dudit cylindre (111) formant un conduit d'admission d'air (111b), ledit cylindre (111) comprenant un premier cylindre (1111) et un deuxième cylindre (1112), ledit tamis de filtrage (113) étant agencé entre ledit premier cylindre (1111)et ledit deuxième cylindre (1112), ledit composant chauffant (112) étant agencé dans ledit premier cylindre (1111), ledit deuxième cylindre (1112) servant à placer un produit de tabac, ledit composant chauffant (112) et ledit circuit imprimé (130) étant connectés électriquement.

3. Une cigarette électronique selon la revendication 2, **caractérisée en ce que**, ladite extrémité de captage de température (121) est agencée dans ledit conduit d'admission d'air (111b), et est en outre proximale de ladite ouverture d'admission d'air (111c) ; ou
ladite extrémité de captage de température (121) est agencée dans ledit cylindre (111), et est en outre agencée à un endroit dudit deuxième cylindre (1112) proximal dudit composant chauffant (112).

4. Une cigarette électronique selon la revendication 1, **caractérisée en ce que**, ledit corps de cigarette (210) comprend en outre un composant chauffant (212) et un tubage (213), ledit composant chauffant (212) étant contenu dans la cavité contenante (211a) dudit cylindre (211), ledit composant chauffant (212) et ledit circuit imprimé (230) étant connectés électriquement, ledit tubage (213) étant emmanché sur l'extérieur dudit cylindre (211), ledit conduit d'admission d'air (214) étant agencé à une extrémité dudit cylindre (211) dotée dudit composant chauffant (212).

5. Une cigarette électronique selon la revendication 4, **caractérisée en ce que**, ladite extrémité de captage de température (221) est agencée dans ledit conduit d'admission d'air (214).

6. Une cigarette électronique selon la revendication 1, **caractérisée en ce que**, ledit cylindre (311) et ledit circuit imprimé (330) sont connectés électriquement, ledit conduit d'admission d'air (313) est agencé à une extrémité dudit cylindre (311), l'extrémité de captage de température (321) dudit élément capteur de température (320) est agencée dans ledit conduit d'admission d'air (313) ; ou ledit corps de cigarette (410) comprend en outre un manchon (412), ledit manchon (412) est agencé à l'extérieur dudit cylindre (411), ledit cylindre (411) et ledit circuit imprimé (430) étant connectés électriquement, ledit manchon (412) et ledit cylindre (411) formant un conduit d'admission d'air (411b), l'extrémité de captage de température (421) dudit élément capteur de température (420) est agencée dans ledit conduit d'admission d'air (411b), et est en outre proximale de ladite ouverture d'admission d'air (411c).

7. Une cigarette électronique selon la revendication 1, **caractérisée en ce que**, ledit corps de cigarette comprend en outre un composant chauffant (520), ledit composant chauffant (520) étant contenu dans ladite cavité contenante (510a), une extrémité dudit conduit d'admission d'air (530) étant emmanchée sur une extrémité dudit cylindre (510), ladite extrémité de captage de température (541) étant agencée dans ledit conduit d'admission d'air (530) à proximité d'une extrémité dudit composant chauffant (520).

8. Une cigarette électronique selon la revendication 7, **caractérisée en ce que**, la paroi latérale dudit conduit d'admission d'air (530) est dotée d'un trou communiquant (530a), ladite extrémité de captage de température (541) s'étirant via ledit trou communiquant (530a) dans ledit conduit d'admission d'air (530) et étant proximale de la partie terminale dudit composant chauffant (520).

9. Une cigarette électronique selon la revendication 8, **caractérisée en ce que**, ledit conduit d'admission d'air (530) comprend un premier conduit (531) et un deuxième conduit (532), ledit premier conduit (531) et ledit deuxième conduit (532) étant agencés selon un angle prédéfini, ledit premier conduit (531) étant emmanché sur une extrémité dudit cylindre (510), une extrémité dudit deuxième conduit (532) étant agencée sur l'autre extrémité dudit premier conduit (531) et communiquant avec ledit premier conduit (531), ledit trou communiquant (530a) étant agencé sur la paroi latérale dudit premier conduit (531) ; ou
ledit conduit d'admission d'air (530) comprend un premier conduit (531) et un deuxième conduit (532), ledit premier conduit (531) et ledit deuxième conduit (532) étant agencés selon un angle prédéfini, ledit premier conduit (531) étant emmanché sur une extrémité dudit cylindre (510), une extrémité dudit deuxième conduit (532) étant agencée sur l'autre extrémité dudit premier conduit (531) et communiquant avec ledit premier conduit (531), ledit trou communiquant (530a) étant agencé sur la paroi latérale dudit deuxième conduit (532).

10. Une cigarette électronique selon la revendication 7, **caractérisée en ce que**, ledit cylindre (510) comprend en outre un corps principal (513) et une enveloppe chauffante (512), ledit conduit d'admission d'air (530) et ladite enveloppe chauffante (512) étant respectivement emmanchées sur les deux extrémités dudit corps principal (513), un trou communiquant (530a) étant agencé sur la paroi latérale de ladite enveloppe chauffante (512), ladite extrémité de captage de température (541) dudit élément capteur de température (540) s'étirant dans ladite enveloppe chauffante (512) via ledit trou communiquant (530a) et étant proximale de la partie terminale dudit composant chauffant (520).

11. Une cigarette électronique selon la revendication 10, **caractérisée en ce que**, elle comprend en outre deux tamis de filtrage (511), lesdits deux tamis de filtrage (511) étant contenus dans ledit cylindre (510), ladite extrémité de captage de température (541) étant agencée entre lesdits deux tamis de filtrage (511).

12. Une cigarette électronique selon la revendication 1, **caractérisée en ce que**, ledit cylindre (610) est un cylindre chauffant, une extrémité dudit conduit d'admission d'air (620) étant emmanchée sur une extrémité dudit cylindre chauffant, un trou communiquant (620a) étant agencé sur la paroi dudit conduit d'admission d'air (620), ladite extrémité de captage de température (631) s'étirant via ledit trou communiquant (620a) dans ledit conduit d'admission d'air (620) et étant proximale dudit cylindre chauffant.

13. Une cigarette électronique selon la revendication 12, **caractérisée en ce que**, ledit conduit d'admission d'air (620) comprend un premier conduit (621) et un deuxième conduit (622), ledit premier conduit (621) et ledit deuxième conduit (622) étant agencés selon un angle prédéfini, ledit premier conduit (621) étant emmanché sur une extrémité dudit cylindre (610), une extrémité dudit deuxième conduit (622) étant agencée sur l'autre extrémité dudit premier conduit (621) et communiquant avec ledit premier conduit (621), ledit trou communiquant (620a) étant agencé sur la paroi latérale dudit premier conduit (621) ; ou
ledit conduit d'admission d'air (620) comprend un premier conduit (621) et un deuxième conduit (622), ledit premier conduit (621) et ledit deuxième conduit (622) étant agencés selon un angle prédéfini, ledit premier conduit (621) étant emmanché sur une extrémité dudit cylindre (610), une extrémité dudit deuxième conduit (622) étant agencée sur l'autre extrémité dudit premier conduit (621) et communiquant avec ledit premier conduit (621), ledit trou communiquant (620a) étant agencé sur la paroi latérale dudit deuxième conduit (622).

14. Un procédé de détection du nombre d'aspirations de cigarette électronique, comprenant :
l'électrification du corps de cigarette, afin de préchauffer l'air présent dans le corps de cigarette jusqu'à ce que la température interne du corps de cigarette atteigne une valeur de température prédéfinie ;
Lorsque aucune aspiration n'est effectuée, la valeur de température détectée par l'extrémité de captage de température de l'élément capteur de température constitue la première valeur de température ;
Lorsqu'une aspiration est effectuée, l'air extérieur pénètre le conduit d'admission d'air via l'ouverture d'admission d'air pour entrer dans le cylindre, et forme un flux d'air s'écoulant dans le conduit d'admission d'air, lorsque le flux d'air passe par l'extrémité de captage de température de l'élément capteur de température, la température détectée par l'extrémité de captage de température constitue la deuxième valeur de température, ladite première valeur de température et ladite deuxième valeur de température présentant un écart de température ; et
le circuit imprimé reçoit l'écart de température produit entre la première valeur de température et la deuxième valeur de température détectées par ledit élément capteur de température, le convertit en signal inducteur et obtient le nombre d'aspirations en détectant le signal inducteur.

15. Le procédé selon la revendication 14, **caractérisé en ce que**, ladite première valeur de température est inférieure à ladite deuxième valeur de température ; ou ladite première valeur de température est supérieure à ladite deuxième valeur de température.

16. Le procédé selon la revendication 14, **caractérisé en ce que**, ladite électrification du corps de cigarette afin de préchauffer l'air présent dans le corps de cigarette jusqu'à ce que la température interne du corps de cigarette atteigne une valeur de température prédéfinie, comprend l'électrification du composant chauffant, une fois ledit composant chauffant électrifié il produit une chaleur, la chaleur préchauffe l'air à la périphérie dudit composant chauffant ; ou, ladite électrification du corps de cigarette afin de préchauffer l'air présent dans le corps de cigarette jusqu'à ce que la température interne du corps de cigarette atteigne une valeur de température prédéfinie, comprend l'électrification du cylindre, après électrification, ledit cylindre chauffe directement de manière conductrice le produit de tabac placé dans ladite cavité contenante.
